Europäisches Patentamt

European Patent Office

Office européen des brevets

(11) Publication number : **0 583 142 A2**

(12) **EUROPEAN PATENT APPLICATION**

(21) Application number : 93306223.4

(22) Date of filing : 06.08.93

(51) Int. Cl.⁵ : **C12N 7/02, A61K 39/29, A61K 35/76**

(30) Priority : **10.08.92 US 926873**

(43) Date of publication of application :
**16.02.94 Bulletin 94/07**

(84) Designated Contracting States :
**AT BE CH DE DK ES FR GB GR IE IT LI LU NL PT SE**

(71) Applicant : **MERCK & CO. INC.**
**126, East Lincoln Avenue P.O. Box 2000**
**Rahway New Jersey 07065-0900 (US)**

(72) Inventor : **Junker, Beth**
**1009 Cranford Ave.**
**Westfield, NJ 07090 (US)**
Inventor : **Lewis, John A.**
**2610 Skippack Pike**
**Norristown, PA 19403 (US)**
Inventor : **Oliver, Cynthia Newell**
**12220 Ambleside Drive**
**Potomac, MD 20854 (US)**
Inventor : **Orella, Charles J.**
**646 Store Road**
**Harleysville, PA 19438 (US)**

Inventor : **Sitrin, Robert D.**
**237 Emerson Drive**
**Lafayette Hill, PA 19444 (US)**
Inventor : **Aboud, Robert A.**
**705 Elmway Circle**
**Blue Bell, PA 19422 (US)**
Inventor : **Aunins, John G.**
**2069 Dogwood Drive**
**Scotch Plains, NJ 07090 (US)**
Inventor : **Buckland, Barry C.**
**626 Boulevard**
**Westfield, NJ 07090 (US)**
Inventor : **Dephillips, Peter A.**
**577 Hidden Valley Road**
**King of Prussia, PA 19406 (US)**
Inventor : **Hagen, Anna J.**
**4 Belmont Square**
**Doylestown, PA 18901 (US)**
Inventor : **Hennessey Jr., John P.**
**114 Fox Hollow Road**
**Dublin, PA 18917 (US)**

(74) Representative : **Cole, William Gwyn et al**
**European Patent Department Merck & Co.,**
**Inc. Terlings Park Eastwick Road**
**Harlow Essex CM20 2QR (GB)**

(54) **Hepatitis A virus vaccine.**

(57) A complete, commercial scale highly reproducible process for producing stable hepatitis A virus (HAV) comprises optimized growth, harvest, and antigen purification conditions, to yield a >95% pure HAV on the basis of protein. Further processing steps yield an inactivated HAV vaccine formulation which is immunogenic, well tolerated, and efficacious in protection against infection by virulent HAV. The product content of protein, carbohydrate, lipid, DNA and RNA is characterized.

FIG.1

BACKGROUND OF THE INVENTION

The field of this invention is the production of a highly purified hepatitis A virus vaccine.

In 1973, Feinstone et al., [Science 182, p 1026] identified the etiologic agent of infectious hepatitis, later known as hepatitis A virus, using immune electron microscopy.

In vitro culture of hepatitis A virus (HAV) was first reported by Provost et al [P.S.E.B.M. 160, p213, 1979] according to a process whereby liver from HAV infected marmosets was used as an inoculum for liver explant culture and fetal rhesus kidney (FRhk6) cell culture [U.S. Patent 4,164,566]. In a later invention, direct inoculation of a HAV which had not been previously passaged through a subhuman primate was successfully used to initiate in vitro propagation of HAV [Provost et al., P.S.E.B.M. 167, p201 (1981); U.S. Patent 5,021,348].

From this work, attenuation of HAV through in vitro culture was demonstrated. In addition, it was demonstrated that upon repeated passage in vitro, HAV cultures became more productive and replication rate accelerated as the virus became adapted to the cultured cells. A further development was the demonstration of protective efficacy in subhuman primates of both the live attenuated virus [Provost; et. al., J. Med Viol. 20, p165 (1986)] and the formalin inactivated HAV [U.S. Patent 4,164,566; U.S. Patent 5,021,348; Provost et. al., in Viral Hepatitis and Liver Disease, p83-86, 1988 - Alan R. Liss, Inc.]. From the foregoing work, it has become clear that either an inactivated or attenuated, immunogenic HAV are possible vaccine candidates. However, a reproducible, commercial scale process for production of high purity antigen is needed if a safe HAV vaccine is to be commercially available for use in humans.

Various methods have been described to partially purify whole HAV virions for investigation and initial virion characterization. See, for example, Hombeck, C.L. et al., Intervirology 6; 309-314 (1975); Locarnini, S.A. et al., Intervirology 10; 300-308 (1978); Siegl, G. et al., J. Virol. 26, 40-47 (1978); Siegl, G. et al., J. Gen. Virol. 57, 331-341 (1981); Siegl, G. et al., Intervirology 22, 218 (1984); Hughes, J.V. et al., J. Virol. 52, 465 (1984); and Wheeler, C.M. et al., J. Virol, 58, 307 (1986).

All of these methods employ unwieldy, impractical and excessively expensive steps, such as sucrose gradient centrifugation or CsCl-density gradient centrifugation. Furthermore, these processes have been adapted to relatively small production scale, and, although some workers have reported that their product was highly purified, careful analysis of the work demonstrates that insufficient parameters were tested to support a claim for any absolute level of purity. Thus, Lewis et al., [European Patent Application 0302692 published on February 8, 1989] disclosed a process comprising (a) HAV infected cell disruption, extraction and concentration; (b) flow through anion exchange chromatography at high salt concentration to remove nucleic acid contaminants; and (c) gel filtration chromatography. In later work, Lewis et al [European Patent Application 0468702, published on July 18, 1989] modified this process by including an ion exchange adsorption/desorption step to remove contaminating carbohydrate. In both of these published works, the scale at which HAV was produced was relatively small and the problems addressed by the instant invention of large-scale, commercial scale-up were not encountered. In addition, the purity of the product was assessed by coomassie or silver stain of gelelectrophoresed product. This assay method provides one measure of protein purity, but it is not quantitative, and it provides no information as to the presence or absence of other contaminants such as lipid, carbohydrate, nucleic acids, or low molecular weight organics.

In both the EPO468702A2 and EPO302692A2 publications, it was stated that prior methods for harvesting and purifying HAV employ one or more steps which are likely to prevent approval by the Food and Drug Administration, by virtue of their use of detergents or exogenous enzymes. In the present invention, both detergents and exogenous enzymes are employed, but the invention discloses methods for removal of these useful reagents, and demonstrates that the product contains less than the limit of detection for these added substances, while being as pure or more pure than HAV products reported in the published literature.

Likewise, Moritsugu et al., [European Patent Application 0339668, published on November 2, 1989], described a process comprising

(a) solubilization, extraction, concentration, DNase/RNase, and proteinase treatment;

(b) Sucrose density fractionation (which separated RNA containing and empty capsids); and

(c) Gel filtration chromatography.

Once again, this process was limited in the scale of production, particularly in regard to the sucrose density fractionation step. In addition, purity of product is difficult to assess and is not reported in any absolute sense in that publication.

Lino et al [Vaccine, vol #10, p5-10 (1992)] reported on enhancements to the Moritsugu process. The product of their process was assayed by SDS-polyacrylamide gel electrophoresis and densitometry to show that there was less than 1.9% of residual exogenous protein in their purified HAV. They also concluded that there was less than 5 pg of residual host cell nucleic acid in 0.5 μg of their purified HAV. However, because of the limitations of their production process, large scale preparation of HAV is impractical, and absolute purity by multiple parameters was not reported.

In another report, Kusov et al [Vaccine, 9, 540 (1991)] cultured HAV in Vero-like cells, and purified the virus by organic extraction, DNase treatment, and chromatography on porous silica beads. Again, there is no indication of absolute purity of the product, nor is there any indication of scalability.

In spite of the uncertainty in the published literature regarding HAV vaccine purity, it is possible to compare vaccine products based on relative antigenicity (see Table 1, Example 11), which shows the comparative amounts of HAV in various products reported by their manufacturer, as compared with the product of the instant invention.

The product of this invention is an immunogen with absolute purity characteristics described in this disclosure representing a level of HAV purity and scale of production heretofore only aspired to. This invention comes a long way from the small scale, pilot level processes reported for making HAV vaccine, making the commerical production of a reproducibly high-quality, highly-purified, safe HAV vaccine a reality for the first time.

## SUMMARY OF THE INVENTION

A process, comprising culture of HAV in a large scale stationary surface bioreactor, detergent harvest of the virus, nuclease treatment, concentration, organic solvent extraction, ion exchange chromatography and gel filtration chromatography, provides a reproducible, commercial scale means of producing a stable HAV vaccine product with an HAV protein purity of >95%. Levels of DNA, carbohydrate, RNA, and lipid are characterized for the vaccine.

## BRIEF DESCRIPTION OF THE DRAWINGS

Fig. 1. A composite flow diagram of the major processes used for Hepatitis A virus vaccine production for clinical trials. The left arm represents a prior art method used for Phase I/II clinical trials which utilized a roller bottle process. The novel process used for the Monroe efficacy study (New England J. of Med., 327: 453-457 1992) is on the right arm and the Manufacturing process of this invention is presented in the center.

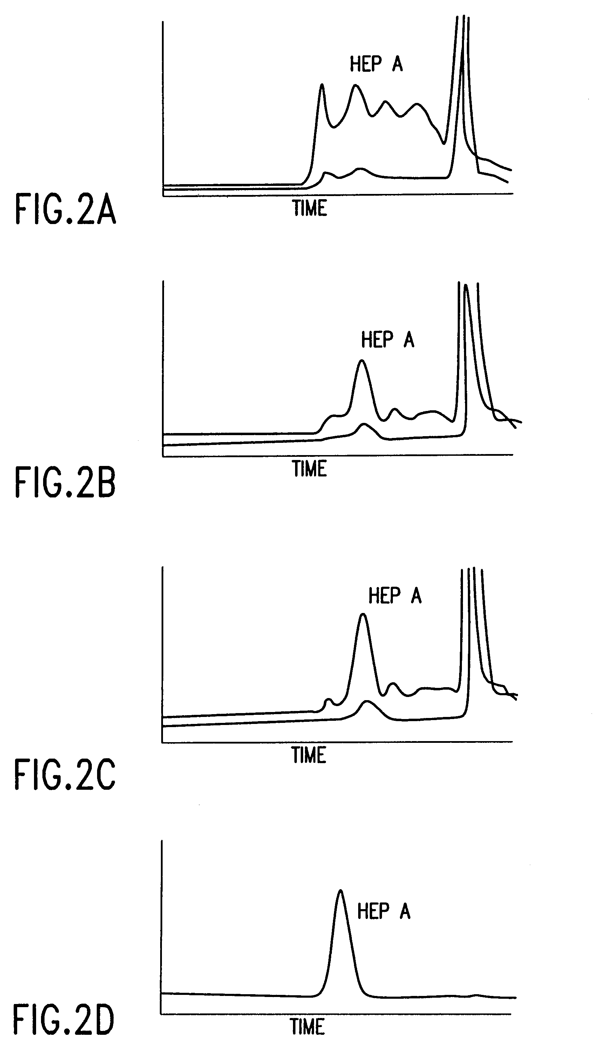

Fig. 2. Analytical sizing in-process assay using a HPSEC TSK PW 4000 column eluted with phosphate buffered saline, PBS. This assay has been calibrated against high molecular weight standards and exhibits a linear area response with Hepatitis A virus dilution. The assay is particularly useful in the latter stages of purification. Four chromatograms are presented here from the last four steps of the Manufacturing process. The chromatography is monitored by dual UV detection at 214 nm (upper tracing) and 260 nm (lower tracing), respectively. Using this assay a peak corresponding to Hepatitis A virus is first visible after resuspension of the PEG pellet (Panel A). The chloroform extraction step removes much of the high molecular weight UV absorbing material (Panel B). The anion exchange chromatography serves to concentrate the virus (Panel C) and the preparative size exclusion chromatography yields a highly purified virus preparation (Panel D). The purified Hepatitis A virus exhibits poor absorbance in the 260 or 280 nm range so only the 214 nm tracing is presented. Under the conditions of this assay, the HAV product is a single, symmetric peak and therefore the assay provides a measure of HAV purity, independent of analysis by SDS PAGE.

Fig. 3. MRC-5 cell growth on glass, polystyrene, and 316 stainless steel. Cell surface concentration is plotted as a function of time. Cells were inoculated on coupons of each material at a density of approximately 10,000/cm$^2$ and a coupon was sacrificed daily for cell counts. Cells on glass coverslips and 316 stainless steel coupons were cultivated in 150 cm$^2$ petri dishes containing 90 mL of medium (0.6 mL/cm$^2$); cells grown in 25 cm$^2$ T-flasks were cultivated with 7.5 mL of medium (0.3 mL/cm$^2$).

Fig. 4. MRC-5 cell growth and residual glucose concentration for cells grown on 316 stainless steel coupons. Cell surface concentration (cells/cm$^2$) and glucose concentration (mg/cm$^2$) are plotted as a function of time (d) for cells grown on 25 cm$^2$ stainless coupons.

Fig. 5. Light micrograph of MRC-5 cells growing on stainless steel gauze. Cells were inoculated at 5,000 - 10,000 cells/cm$^2$ and cultivated in excess of 2 weeks. Magnification 40X.

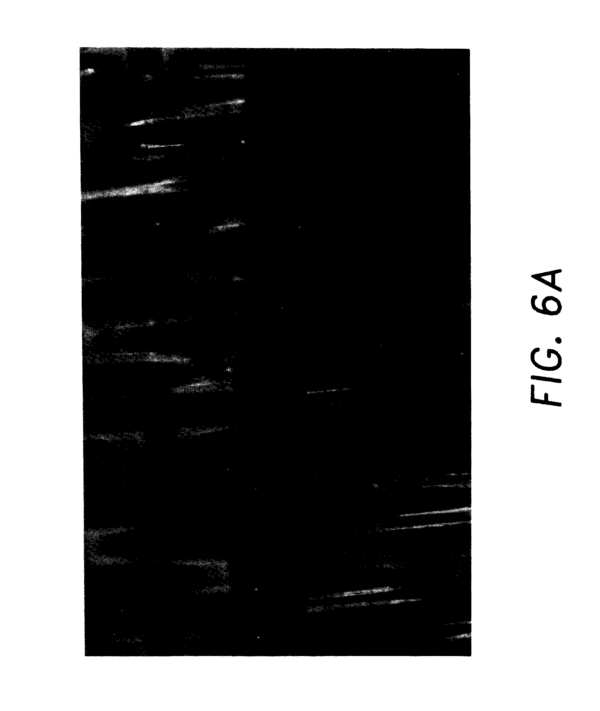

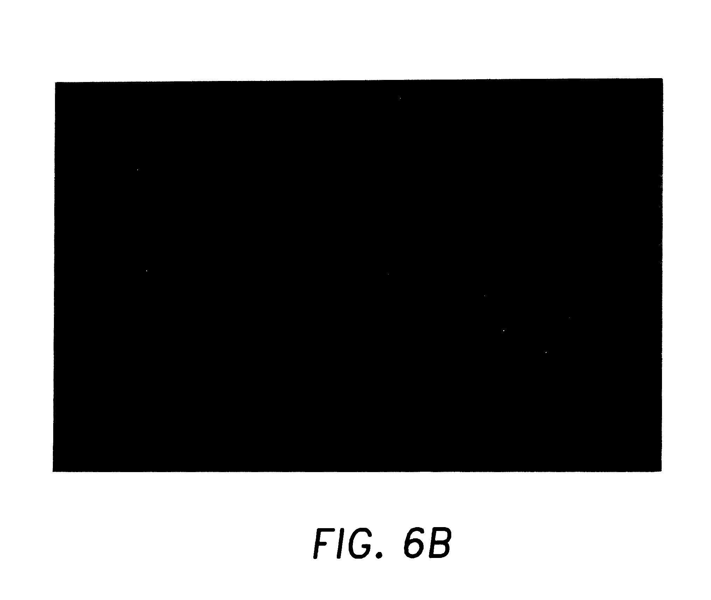

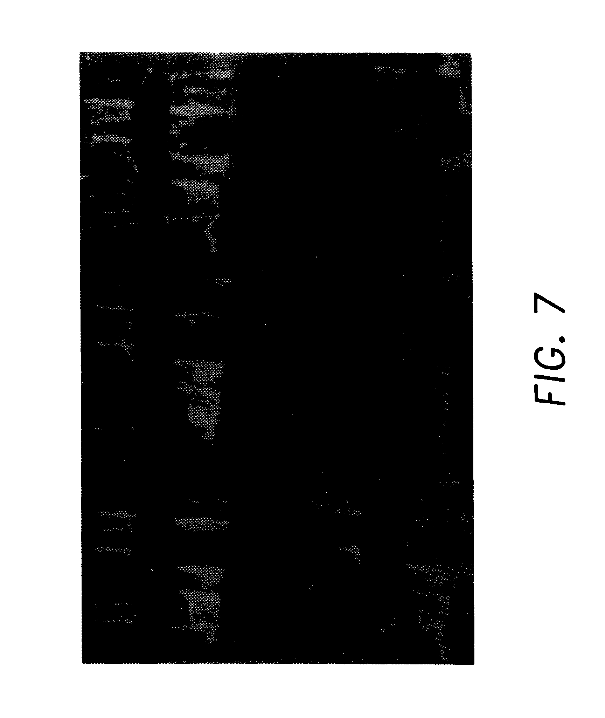

Fig. 6. Viable and non-viable staining of MRC-5 cells grown on 316 stainless steel gauze. Cells were stained with fluorescein diacetate (FDA) and ethidium bromide (EB) according to the protocol described in the Material and Methods section of Example 14, and then viewed using a scanning laser confocal microscope; the material was scanned in 57 μm increments. Figure 6A corresponds to fluorescence from fluorescein, and indicates viable cells. Figure 6B is fluorescence due to ethidium bromide staining of DNA, demonstrating the near complete viability of the cells. The positive control for ethidium bromide is shown in Figure 7.

Fig. 7. Ethidium bromide stain of non-viable MRC-5 cells. Cells grown on 316 stainless steel gauze were made non-viable by treatment with 100% methanol. After rinsing well with PBS and allowing to dry cells were

EP 0 583 142 A2

stained with FDA/EB to serve as a negative control for FDA and a positive control for ethidium bromide (pictured). No fluorescence due to fluorescein production was observed for the negative control.

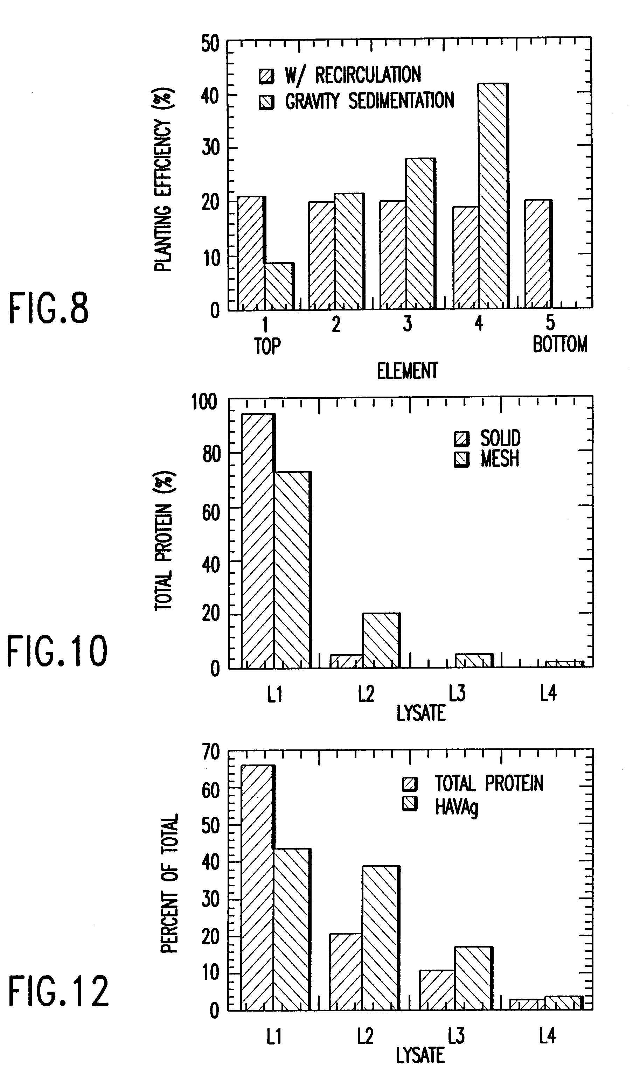

Fig. 8. Planting efficiency is plotted for each element in a gauze static mixer reactor for protocols employing gravity sedimentation, or medium recirculation. Elements are numbered 1 through 5, with 1 being the top element and 4 or 5 being the bottom element (4 elements were used in gravity sedimentation experiments and 5 for recirculation experiments). The planting efficiencies presented here are the percent of the total planted; overall planting efficiencies were on the order of 90%. Data for recirculation is an average of 3 experiments while that for gravity sedimentation is from a single experiment. Recirculation velocities were approximately 6 cm/h.

Fig. 9. Glucose uptake rates (GUR) as a function of time for reactors containing solid titanium and gauze 316 stainless steel elements. GUR on a per $cm^2$ basis ($\mu g/cm^2$-d) against time (d) is shown in Figure 9A while GUR on a per reactor volume basis is plotted in Figure 9B. The data in Figure 9A is dependent on the estimated surface areas of the elements while Figure 9B embodies no estimation since both reactors are the same volume.

Fig. 10. The percent of total protein observed in successive lysates for the solid and gauze static mixers. The protocols for lysates 1-4 are described in the text. By microscopic observation, solid elements were free of cell debris while the gauze elements contained considerable cell debris after the fourth lysis.

Fig. 11. Glucose uptake rates as a function of time for a 316 stainless steel gauze static mixer reactor. The reactor was infected with an MOI of 1 on day 7 and harvested on day 28.

Fig. 12. The percent of total protein and HAVAg in successive lysates for the gauze static mixer shown in Figure 11. The protocols for lysates 1 - 4 are described in the text. The total protein of lysate 1 was artificially high due to inefficient PBS washes.

Fig. 13. The effect of MOI on cell growth and glucose consumption. Cells were infected with an MOI of 0, 1, and 10 with HAV CR326F P28. Differences in cell density (13A) and residual glucose (13B) are not observed until 8 days post-infection for this system. A complete medium refeed was performed on day 8.

Fig. 14. Cellular protein measured against a BSA standard versus number of cells. The best fit line is described by the equation: $Y(10^4) = -0.09 + 0.42X$, r = 0.99. This Figure demonstrates that a single curve provides a means to measure MRC-5 cell number by total protein, regardless of the "state" of the cells.

Fig. 15. Figure 14 is replotted, indicating the time of each data point.

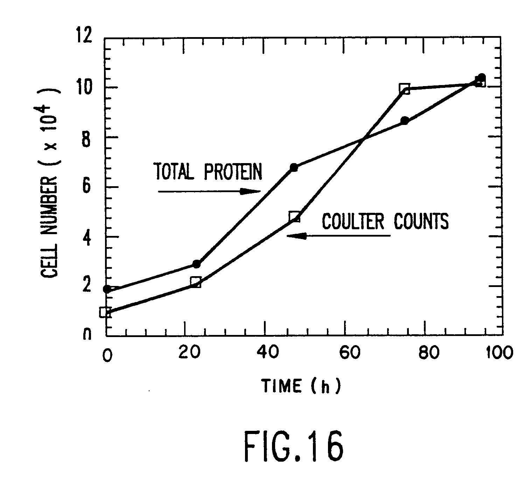

Fig. 16. Conversion of total protein to a cell number closely resembles the cell density obtained using a Coulter Counter. It is believed that discrepancies are due to the small volumes of lysis buffer used in harvesting the T-25s (2X 0.08 $mL/cm^2$ was used for consistency).

Fig. 17. Purification across Solvent Extraction Step, HPSEC Profiles (manufacturing consistency lots)

Fig. 18. Purification across Solvent Extraction Step, HPSEC Profiles (piloting lots)

Fig. 19. Effect of Mixing Time on Hep A/Impurity Ratio

Fig. 20. Effect of Mixing Time on Hep A and Impurity Areas (Shaker, 50 mL bottles, 6 minutes)

Fig. 21. Effect of Mixing Time on Hep A and Impurity Areas (Shaker, 500 mL bottles, 2 minutes)

Fig. 22. Effect of Mixing Time and Tube Volume on Hep A and Impurity Areas (Shaker)

Fig. 23. Effect of Solvent/Aqueous Ratio on Hep A/Impurity Ratio

Fig. 24. Effect of Solvent/Aqueous Ratio Hep A and Impurity Areas (Shaker)

Fig. 25. Effect of Mixing Time and Type on Hep A/Impurity Ratio

Fig. 26. Hepatitis A Sample Stability determined by HPSEC

Fig. 27. Effect of Salt on Hepatitis A Solubility (At 4 Degrees)

Fig. 28. Effect of Salt on Hepatitis A Solubility (At 4 Degrees)

Fig. 29. Molecular weight calibration curve, PW4000x1.

Fig. 30. Log Hepatitis A peak area v. log hepatitis A concentration.

Fig. 31. Hepatitis A calibration curves.

Fig. 32. Comparison of the Filtered Lysate and Nuclease Treated Lysate samples, UV at 214 nm

Fig. 33. Comparison of the Filtered Lysate and Nuclease Treated Lysate samples. UV at 260 nm.

Fig. 34. PEG Pellet Resuspended sample. UV at 214 nm.

Fig. 35. Chloroform Extracted Aqueous Phase sample from Rx2011008. UV at 214 nm.

Fig. 36. Anion Exchange Product sample from Rx2009854. UV at 214 nm

Fig. 37. Size Exclusion Chromatography Product. UV at 214 nm.

Fig. 38. SEC Product sample from Rx2011008. UV at 214nm. Retained peak visible at about 49 minutes.

Fig. 39. A correlation of EIA and HPSEC results for samples from 11 lots.

Fig. 40. Area percent values for 4 samples from 13 Hepatitis A lots.

4

DETAILED DESCRIPTION OF THE INVENTION

The commercially adaptable purification process of the present invention encompasses the purification of any hepatitis A virus (HAV), whether derived from attenuated or virulent HAV, produced in any cell strain, line, or culture susceptible to infection by HAV. In addition, all HAV capsids, whether empty (without viral nucleic acids) or complete (containing viral nucleic acids) are encompassed within the process of the present invention.

HAV at passage twenty-eight (P28CR326F'; for the taxonomy of variants arising from CR326, such as strain CR326F and CR326F', and the relationship of this nomenclature to absolute passage level, see Provost et al, J. Med. Virol., 20:165-175, 1986) was used to infect MRC-5 cells in this invention, for merely illustrative purposes, and the production material was cultured at P29. P28CR326F' is an attenuated HAV strain. Other strains of HAV are encompassed by this invention, including HAV strains that can be attenuated by conventional techniques. Other suitable cell lines for HAV propagation include Vero, FL, WI-38, BSCI, and FRhK6 cells. These and other systems for HAV propagation in cell cultures are discussed in Gerety, R.J. "Active Immunization Against Hepatitis A," in Gerety, R.J. (ed.) Hepatitis A Academic Press 1984, pp. 263-276; and Ticehurst, J.R., Seminars in Liver Disease 6, 46-55 (1986). In principle, any cell line such as any human diploid fibroblast cell line, can serve as a host cell for HAV provided that it is susceptible to HAV infection. A preferred cell line for vaccine production is MRC-5.

The commercial scale process of this invention comprises the steps of:

(a) Culturing a large quantity of Hepatitis A virus (HAV) in a suitable culture vessel and medium:

Preferred modes of accomplishing this include, but are not limited to, the use of HAV adapted to growth in human diploid lung cells suitable for vaccine production. WI38 cells or MRC-5 are acceptable. These cells have been grown on microcarriers or as cell sheets in flasks or roller bottles. The process of this invention utilizes large surface area, multilamellar bioreactors such as the NUNC CELL FACTORY, COSTAR CUBE Static Surface Reactor (SSR), or a static mixer reactor such as that described in U.S. Patents 4,296,204, 4,415,670, or as further described herein in a stainless steel, gauze, static surface mixer, depending on the desired scale of production. Thus, in a preferred embodiment of this invention, MRC-5 cells are grown in cell sheets in an 85,000 cm² COSTAR CUBE SSR, or gauze SSR of even greater surface area, infected at a multiplicity of infection (MOI) of HAV sufficient to achieve efficient cell culture infection. An MOI of about 0.01-10 is acceptable. Stock seed is conveniently generated by using HAV from the supernatant fraction of an SSR incubated for about 28 days.

In a preferred embodiment, HAV is cultured on MRC-5 cells grown on stainless steel gauze. (See Example 14). According to this embodiment, a bioreactor is used wherein mesh elements, made from any suitable, nontoxic material on which cells can grow, are used. Such elements may be made from stainless steel, titanium, plastic or similar material which can be provided in a regular three-dimensional array. Preferably 316 stainless steel mesh elements such as are available from Sulzer Biotech Systems (particularly SMV, SMX, E-PACK EX, DX, BX, CY), are used. Each element is comprised of a uniform array of mesh layers on which cells may grow. Each element of regular meshwork is oriented at 0-90° angles to each other in a sterile reactor sytem. The configuration may be a linear column or any other geometric shape adapted to accomodate the mesh elements and which makes good use of volume. Cells are then seeded and allowed to attach to the vast surface area provided by the mesh. Once the cells are well established in culture, they can be fed at a perfusion rate sufficient to maintain nutrient exchange and eliminate waste products. At an appropriate cell density, the cells are infected with HAV as further described below.

The advantage of the mesh elements is that the fibrous bed allows more surface area per unit volume. Geometrically precise mesh allows uniform growth of fibroblasts across the surface, to known and constant depths, allowing control of nutrient delivery. The uniformity of the mesh also allows predictable contact points between fibers to allow rapid cell migration to cover the fiber network. The static mixer configuration controls the proximity of the growth surfaces to each other such that the natural biological growth and "fouling" of the reactor is avoided. This is a serious problem encountered in random-packed bed reactors, where the non-uniformities of the packing allow localized regions of cell growth which then cause a loss of nutrient flow and subsequent cell starvation.

It will be understood that the scope of the present invention encompasses, in addition to the passage 18, P18, p28, p29, p72 of strain CR326F of HAV, any other HAV variant or strain, whether attenuated or virulent. Attenuated variants or strains may be isolated by serial passage in cells, animals, or by other methods. See, for example, Provost, P.J. et al. Proc. Soc. Exp. Biol. Med. 170,8 (1982); Provost, P.J. et al. J. Med. Virol. 20, 165 (1986): U.S. Patent 4,164,566 and 5,021,348 for details on attenuation. The purification methods of the present invention are readily and easily adaptable to attenuated or virulent HAV strains.

The HAV is allowed to replicate in the SSR to a peak of virus production. For attenuated, cell culture adapted strains of HAV such as CR326FP28, this takes from 7-35 days, depending on the initial MOI and cell density during which time nutrient medium is constantly circulated through a loop which provides gas exchange. The cell culture medium may be any medium which supports active growth of MRC-5 cells and HAV replication. Preferably, the culture medium is replenished and removed at a rate between about 0.010 and 0.3 mL/cm$^2$/day. This rate is flexible because the rate of cell mass generation is the total exposure to medium.

Thus, in a preferred embodiment of this invention, MRC-5 cells are grown in cell sheets in a 340,000 cm$^2$ SSR (for example, using four 85,000 cm$^2$ elements) or gauze SSR of even greater surface area, infected at a multiplicity of infection (MOI) of HAV of about 0.1-1.0, and the HAV is allowed to replicate to a peak of viral antigen production. This takes about 28 days, during which time nutrient medium is replenished and circulated through the culture vessel and a loop which provides gas exchange. The culture medium may be any medium which supports growth or maintainance of MRC-5 cells, and HAV replication. We have found that medium comprising a basal medium, such as William's Medium E, supplemented with appropriate growth factors is preferred. As a source of growth factors, we have found that an iron-supplemented calf serum is preferred.

(b) Harvesting the cultured HAV.

The method of harvesting the cultured HAV will be dictated, to some extent, by the geometry of culture vessel. In general, following culture for about 28 days with nutrient replacement, the culture medium is drawn off and the HAV is harvested. In the past, for small-scale propagation of virus, this has been accomplished by scraping followed by freeze thawing and optionally sonicating to optimize release of the cell-bound virus. In the process of this invention, cell bound HAV is liberated into a minimal volume of harvest solution. Preferably, the harvest solution contains a component effective to render the cells permeable to HAV. Such components are known in the art. Preferably, a detergent such as Triton X-100, NP-40, or an equivalent is supplied at the lowest effective concentration possible, to facilitate later removal. Provision of about 0.05-0.5%, and preferably 0.1% Triton X-100 has been found adequate to achieve efficient extraction of HAV from cells cultured in NUNC cell factories, COSTAR cubes, or any other cell sheet culture. The advantage of using detergent extraction, particularly from an SSR such as the COSTAR CUBE is that the geometry of such a culture vessel does not permit harvest by mechanical means, except to the extent that HAV is found in the culture supernatant, which can be drawn off and the HAV concentrated and recovered. However, the proportion of HAV found in culture supernatant is generally only a small fraction of the total HAV recoverable from the cells. Detergent harvest of HAV from cultured cells is highly efficient.

(c) Harvested HAV Treatment.

Once the HAV has been harvested in a substantially cell-free form, from the culture supernatant, from the cells, or both, it is desirable to concentrate the HAV to facilitate down-stream processing and purification. In a prior art process of purifying HAV, where HAV was harvested by mechanical means and where only small quantities of material were being purified, the HAV was extracted with an organic reagent such as methylene chloride, or methylene chloride:isoamyl alcohol (24:1, V/V), followed by concentration of the aqueous phase by addition of a water soluble synthetic polymer, such as polyethylene glycol. It has now been discovered that detergents and exogenous enzymes can be employed in a commerical scale process for preparation of whole HAV vaccine with removal of the added materials to the point where traces are not detectable in the purified product.

In one embodiment of this invention, a detergent, such as Triton X-100, used in HAV harvest, is removed by a concentration/diafiltration, followed by removal of residual detergent, for example with an AMBERLITE nonionic polymeric adsorbent such as XAD. A useful method for showing removal of Triton X-100, down to about 10 μg/ml, has been reported by Humi, W.M., and Miller W.J. [Journal of Chromatography, 559, 337-343 (1991)]. Detergent used in HAV harvest is undetectable by this analysis in the product of this embodiment of the invention. In addition, many other contaminants are removed, yielding a partially purified HAV product.

In another embodiment of the invention, following detergent harvest, rather than concentration/ diafiltration, followed by XAD treatment, a nuclease treatment followed by an anion exchange capture step is employed for concentration.

In this embodiment, preferably a non-specific, high specific activity, and highly homogenous nuclease is used. The nonspecificity ensures cleavage of both DNA and RNA, and the homogenity of the enzyme ensures that only one enzyme is being added, without addition of other proteinaceous contaminants. It is furthermore preferable, in the process of this invention, for the nuclease to be detergent resistant, so that it can be added directly to the detergent harvested HAV. Any of a number of nucleases may meet these criteria. However, BENZONASE, prepared as a recombinant protein by Nycomed Pharma A/S (Denmark), has been discovered to be particularly preferred for this purpose. Addition of between 0.1 μg - 100 μg/L of harvested HAV, and preferably about 10 μg/L, has been found to be acceptable for removal of contaminating free nucleic acid.

Addition of a minimal amount of enzyme is important to facilitate subsequent removal of the enzyme in down-stream processing steps, to yield a product with undetectable levels of residual enzyme (less than about 10 pg/mL).

Since the nuclease treatment occurs in a relatively dilute solution, we have found it convenient to capture the HAV by anion exchange chromatography in a dilute (90 to 150 mM) salt solution, and then elute the HAV by a step addition of about 1.5 column volumes of higher ionic strength ($\geqq$ about 0.3 M) solution. The HAV is substantially concentrated by this capture step and, in addition, most of the detergent, BENZONASE and many other contaminants, flow through the column. Therefore, the HAV that is eluted from the capture column is partially purified. Any of a number of ion exchange resins known in the art may be used for this step, including those listed in EPO468702A2. We have discovered that a particularly preferred resin for this purpose is to use DEAE TOYOPEARL 650M (Tosohaas).

(d) HAV Concentration.

Following HAV harvest and concentration/diafiltration/XAD or nuclease treatment/capture, the HAV is concentrated with a water soluble synthetic polymer effective to concentrate proteins. Polyethylene glycol (PEG), having a molecular weight of between approximately 2,000 daltons and 12,000 daltons, is effective for this purpose. Typically, NaCl is added to the partially purified HAV to a final concentration of between approximately 150 mM and 500 mM. PEG is then added to a final concentration of between approximately 2% (w/v) and 10% (w/v), and preferably about 4%. The resulting 4% PEG precipitated partially purified HAV is centrifuged, the supernatant is discarded and the pellet resuspended for further processing. We have discovered that a major contaminant, is removed in the discarded supernatant of this step. Removal of the protease enhances the yield, stability, and purity of the HAV product through the remainder of the purification. The resuspended PEG pellet is optionally sonicated to assist in solubilization before organic extraction, as described below.

(e) Organic Extraction.

The resuspended, PEG pellet from above is extracted by the addition of a mixture of a halogenated lower alkane containing 1-6 carbons, such as methylene chloride, chloroform, tetrachloroethane, or the like, and an antifoaming agent, such as isoamyl alcohol. The volume-to-volume ratio of halogenated lower alkane to antifoaming agent is between approximately 15:1 and 50:1, and is preferably between about 20:1 and 30:1. Applicants have discovered that organic extraction substantially enhances purity of the final HAV product. Chloroform is superior to methylene chloride for organic extraction at this stage.

Such extraction removes, among other contaminants, lipid and lipid-like substances. Accordingly, the resuspended PEG pellet is diluted with any one of a variety of buffers, including phosphate buffered saline, TRIS, carbonate, bicarbonate, or acetate buffers. Since HAV capsids are stable in mildly acidic conditions, buffers in a slightly acidic pH range are acceptable. One preferred buffer is TNE (10 mM TRIS-HCl, pH 7.5, 150 mM NaCl, 1 mM EDTA). Phosphate may be used in place of TRIS-HCl.

Preferably, the resuspended PEG pellet is organically extracted by the addition of chloroform/isoamyl alcohol (24:1,v/v) and a an organic to aqueous ratio of between about 0.5:1 and 3:1, with vigorous vortexing. This extract is then clarified by centrifugation at 4,000 g for 10 minutes at 20°C. The aqueous phase is reserved, the interface and organic phase are reextracted with a volume of TNE or equivalent buffer equal to between about 0.3 and 0.6 of the original sample volume, and both aqueous phases are combined, yielding extracted PEG pellet.

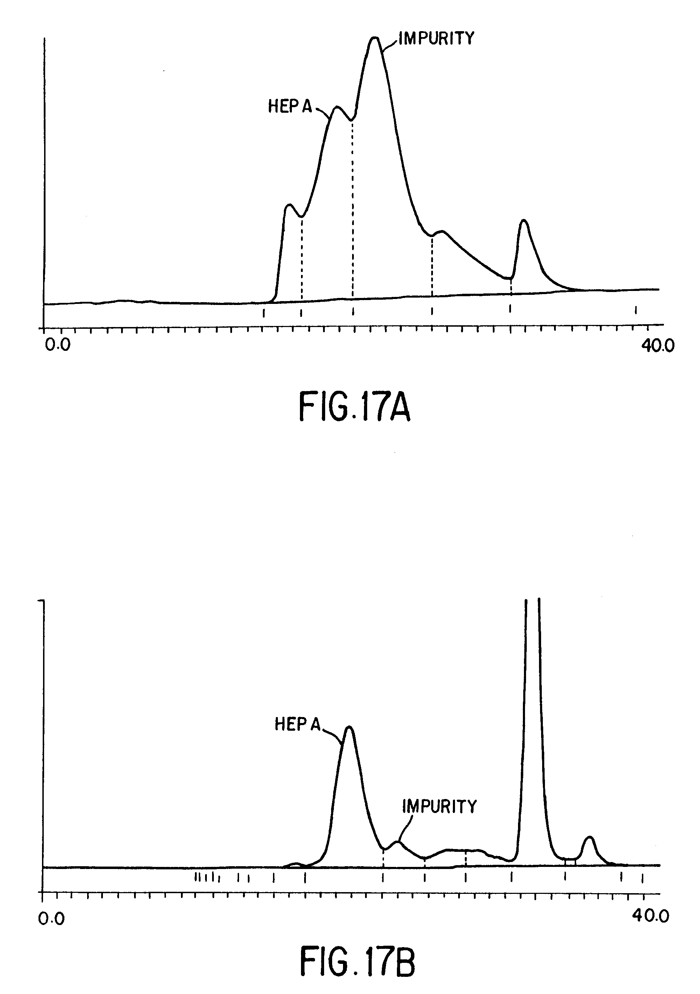

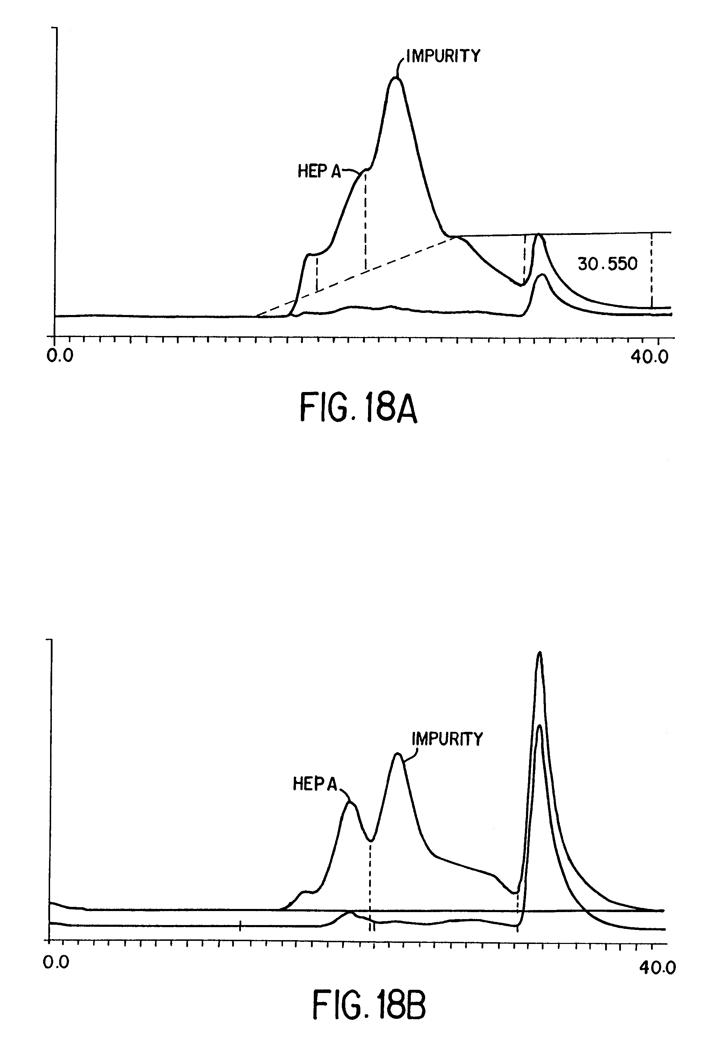

The solvent extraction step plays a key role in the purification process of Hepatitis A by precipitating protein impurities at the interface as well as extracting lipids into the solvent phase. The considerable purification achieved by this extraction during the manufacturing consistency lots can be seen from the HPSEC chromatograms in figure 17, where the impurity peak at 23 min is a useful marker to study impurity removal across this step. In contrast, during the smaller scale piloting lots, a significant proportion of this peak was retained in the extracted product (figure 18). Several variables were investigated in order to understand and control this step upon scale-up: ratio of solvent to aqueous volumes, type of shaking, mixing time and mixing intensity were studied in several tube and bottle sizes (See Example 15).

(f) Ion Exchange chromatography.

The extract from above is subjected to anion exchange chromatography on a resin, gel or matrix. Typical anion exchange matrices include, but are not limited to,

DEAE cellulose;

DEAE agarose;

DEAE Biogel;

DEAE dextran;

DEAE Sephadex;

DEAE Sepharose;

Aminohexyl Sepharose;

Ecteola cellulose;

TEAE cellulose;

QAE cellulose;

mono-Q; or

Benzoylated diethylaminoethyl cellulose.

One preferred anion exchange matrix is DEAE TOYOPEARL 650M (Tosohaas). General background information on ion exchange chromatography can be found, for example, in E. A. Peterson, "Cellulosic Ion Exchangers" in Work, T.S. et al. Laboratory Techniques in Biochemistry and Molecular Biology Volume 2, Part II, pages 223 et seq. North-Holland 1970.

The organically extracted resuspended PEG pellet is diluted, if necessary, with 10 mM TRIS, 1 mM EDTA, pH 7.5, or an equivalent buffer, and NaCl is added to yield a NaCl concentration between approximately 0.3 M and 0.35 M if necessary to remove contaminating free nucleic acid. The HAV passes through the column in filtration mode under these conditions. However, if the HAV preparation was previously treated with nuclease, the organic extract is brought to between 0.1 M and 0.15 M NaCl, with 0.12 M NaCl preferred, in anticipation of the next step.

At the NaCl molarity described above, HAV capsids adhere to the anion exchange matrix while certain remaining contaminants, such as carbohydrates, pass through the matrix. The column is washed with several column volumes of 0.12 M NaCl to further remove remaining contaminants. The HAV capsids are then eluted from the anion exchange column using about one column volume plus the original sample volume of 0.35 M NaCl. Alternatively and preferably, the HAV is eluted by gradient elution up to about 1M NaCl or equivalent salt, with the HAV eluting at about 0.3 M NaCl.

(g) Gel Filtration.

A final step of gel filtration chromatography follows anion exchange chromatography. Typically, Sepharose CL-4B (Pharmacia) is employed, but numerous other types of gel filtration matrices can be substituted. See, for example, Fischer, L., "Gel Filtration Chromatography," in Work, T. S. et al. Laboratory Techniques in Biochemistry and Molecular Biology Elsevier (1980). We have discovered a particularly preferred method for applying size exclusion chromatography to the purification of HAV at this step. This comprises elution of as small a sample volume as possible of the anion exchange purified HAV on tandem columns of TOYOPEARL HW55S and HW65S.

While the particular sequence of chromatographic steps of anion exchange followed by gel filtration are the typical protocols for purifying HAV capsids in this invention, it will be understood that the sequence can be varied. For example, gel filtration may precede the anion exchange step. We have found that the foregoing sequence is preferred.

(h) HAV Precipitation.

A completely unexpected discovery in the course of the large scale production of HAV, relating to solubility of the virus, occurred as a result of the large quantities of HAV produced according to this invention. We have discovered that in the 0.25-0.3M salt concentration at which HAV is eluted from the ion-exchange column, precipitation of the virus occurs if the HAV concentration is above about 10-20 µg/ml.

This was discovered by the HPSEC analysis of representative samples which showed that the Chloroform Extracted Aqueous Phase (AQX) and the Size-Exclusion Product (SEC) were very stable on storage a 4°C; but the Anion Exchange Product (IEX) showed a marked loss of Hepatitis A upon storage at 4°C.

To confirm this finding, a stability study was initiated with a second set of samples. The AQX, IEX Product, and SEC Product were each assayed (in triplicate) by HPSEC immediately upon genesis. The Hepatitis A concentration was then determined by comparison of the resulting peak areas to an SEC Hepatitis A calibration standard. In addition, an aliquot of the IEX Product was diluted 1 in 5 with phosphate buffered saline (0.12M NaCl, 6mM sodium phosphate, pH 7.2) and also quantitated immediately. The stability of these 4 samples at 4°C was monitored by HPSEC over several days.

The initial Hepatitis A concentration and the value obtained 4 to 5 days later for several of these Hepatitis A purification stream samples are given below.

| Sample | Initial Hep A Conc. (ug/ml) | Day 4 Hep A Conc (ug/ml) |
|---|---|---|
| AQX | 23 | 21 |
| IEX Product | 59 | 50 |
| IEX Product diluted 1 in 5 | 12 | 10 |
| SEC Product | 12 | 12 |

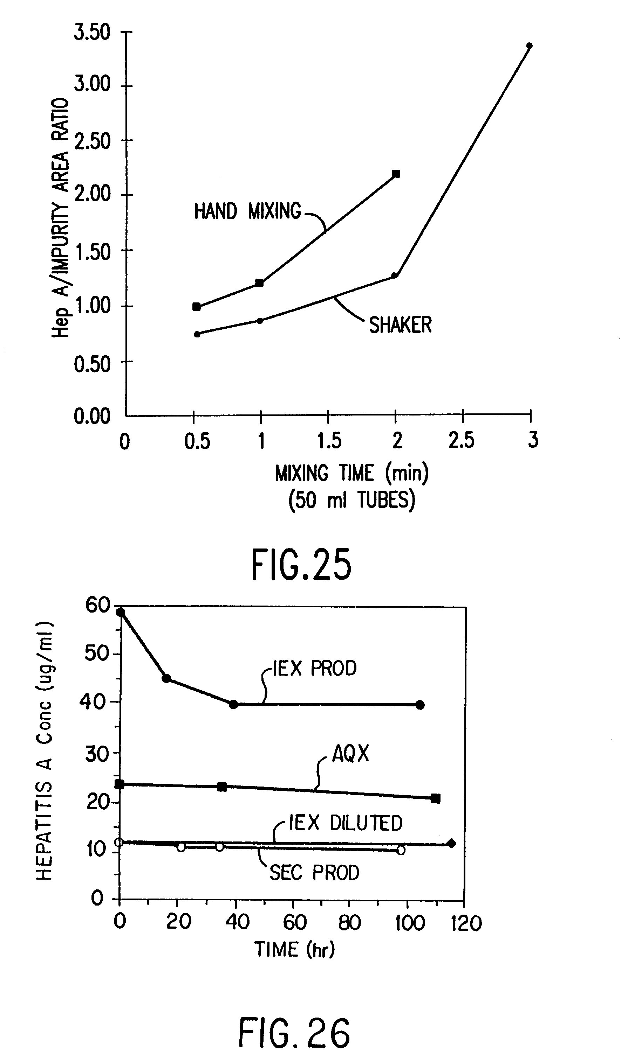

The complete results, including intermediate time points, are shown in figure 26.

As seen with the first lot, the IEX Product shows a marked loss of Hepatitis A, with a loss of approximately 25% occurring on overnight storage (Hep A conc. dropping from 59 to 45 ug/ml).

The diluted IEX Product and the AQX Product show much greater stability, with Hepatitis A losses of only about 10% through the first 100 hours of storage. The SEC Product showed no loss of Hepatitis A, essentially the same result obtained for the first lot.

One difference between these two lots is the formation of a visible precipitate that occurred with the second lot. The presence of precipitate necessitated centrifuging the entire IEX Product prior to the SEC step. This centrifugation resulted in a large loss of precipitated Hepatitis A; the concentration after centrifugation was 29 ug/ml. A yield table calculated from the HPSEC data is shown below.

| Sample | total mg of Hep A | Step Yield | mg of Hep A after sampling |
|---|---|---|---|
| AQX Product | 12.9 | | 11.7 |
| IEX Product | 12.3(initial amt) | 105% | 8.7 |
| Centrifuged IEX Product | 4.2 | 48% | 4.2 |
| SEC Product | 2.0 | 49% | 1.4 |

The unexpectedly low SEC yield suggested that aggregation or precipitation may be occurring on the SEC column. In fact some aggregate was observed by HPSEC. A 7 ml sample of the IEX Product that was chromatographed on a smaller set of tandem SEC columns (immediately after the prep IEX was finished) showed a Hepatitis A recovery of 71%, which is more typical for the step. Because this sample did not suffer the Hepatitis A loss on centrifugation (because it had not yet precipitated) the yield of 71% is about 3 times what was obtained at production scale in going from IEX to SEC Product. The smaller scale tandem SEC resulted in a much greater dilution factor, which may account for the higher yield.

The precipitate observed during purification of these lots which coincided with a loss of virus by HPSEC was unexpectedly found to be soluble in low ionic strength aqueous solution (e.g. 6 mM sodium phosphate), such that the precipitation was reversible. Precipitate was re-dissolved by adding 6 mM sodium phosphate, which gave rapid dissolution. In contrast, resuspension of the precipitate in 120 mM sodium chloride with 6 mM sodium phosphate or in higher ionic strength solutions gave little change. Based on HPSEC retention times, the precipitate that was dissolved in 6 mM sodium phosphate was the same size as monomeric Hepatitis A, while SDS-Page with silver staining indicated that the dissolved precipitate had the same protein bands visible in final SEC product. Based on this data the purification of Hepatitis A has been modfied to include a dilution of the ion-exchange product to prevent the loss of precipitate (See Example 16), such that a 1:1 dilution brings the salt concentration to about 0.15M or below. It is also desirable to achieve an HAV concentration of below 20 μg/ml.

(i) <u>HPSEC Consistency Assay.</u>

A High Performance Size Exclusion Chromatography (HPSEC) assay has been developed to quantitate Hepatitis A concentrations and impurity levels in samples from the Hepatitis A purification process.

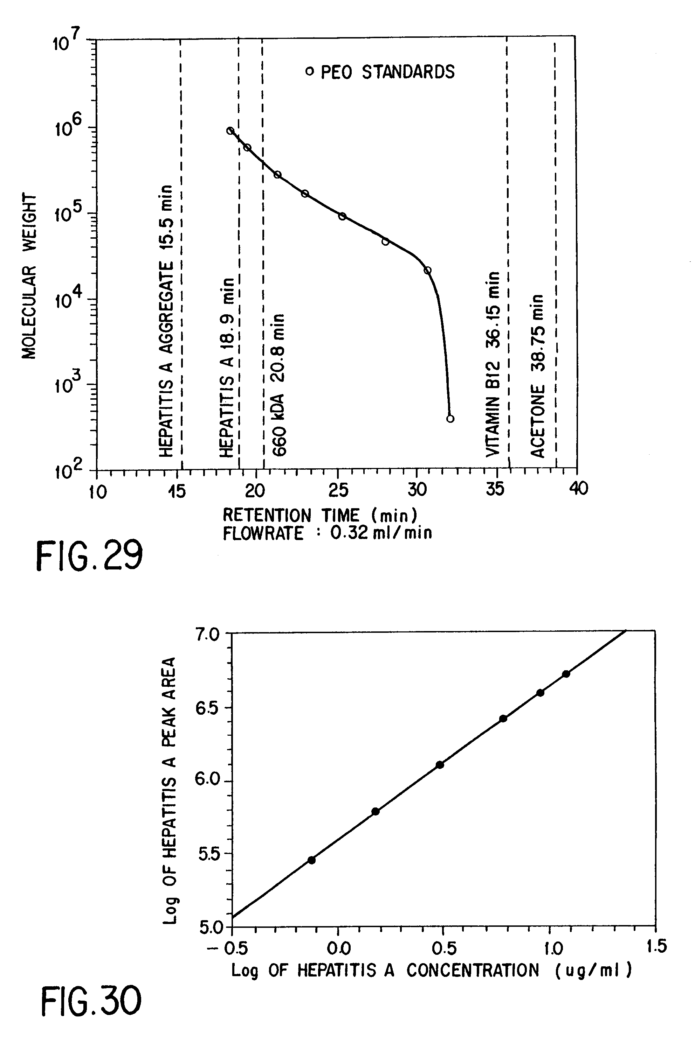

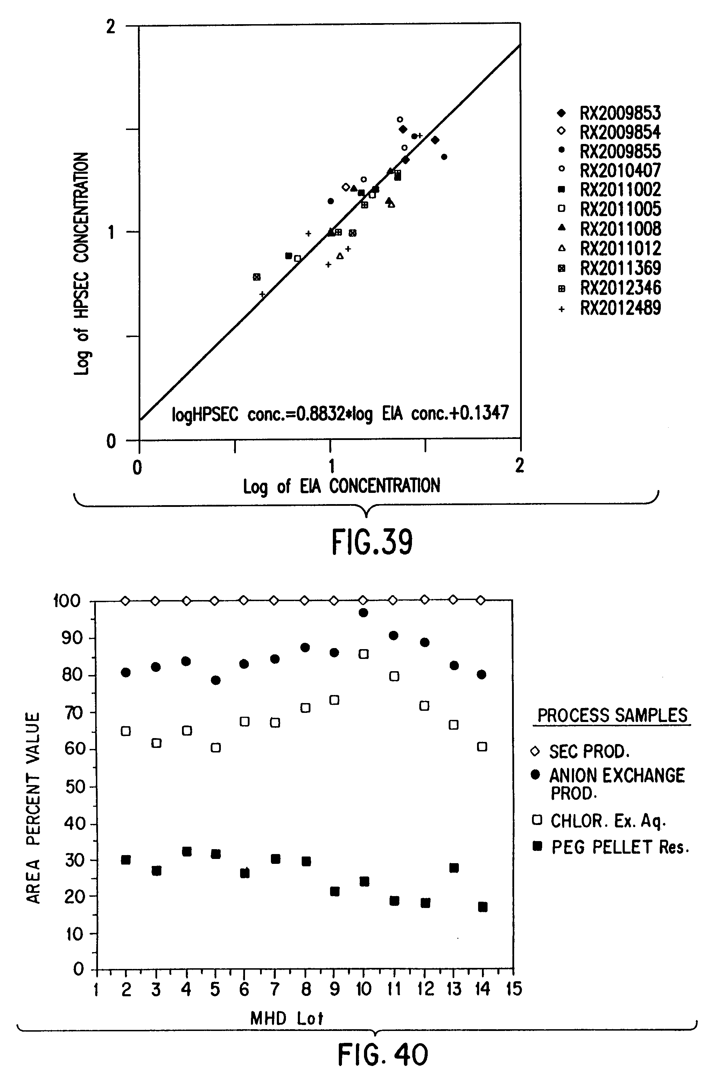

Hepatitis A process samples from a total of 14 lots prepared according to this invention have been analyzed; the resulting database has been used to characterize the Hepatitis A levels and contaminant profile for each of the purification steps. The HPSEC assay conditions, validation data demonstrating the accuracy and precision of the assay, a comparison of HPSEC to EIA data, and results from HPSEC analysis of 10 lots are presented in Example 17 and figures 29-46.

(j) Vaccine Inactivation and Formulation.

Additional processing steps of conventional and well known character are or may be needed to prepare purified HAV capsids for vaccine use. For example, treatment with formalin, sterile filtration and adsorption to carriers or adjuvants are the typical basic steps for preparing a formalin-inactivated vaccine. See, for example, Provost, P.J. et al. Proc. Soc. Exp. Biol. Med. 160, 213 (1979); Provost, P.J. et al. J. Med Virol. 19,23 (1986). HAV can be inactivated by heat, pH changes, irradiation, treatment with organic solvents such as formalin or paraformaldehyde. Typically, HAV inactivation is carried out at a 1/4000 ratio of formalin. We have discovered that the formalin inactivation proceeds more rapidly and efficiently at four times the standard concentration, and a 1/1000 formalin concentration reduces inactivation time by a factor of 4 without adversely affecting immunogenicity. The inactivated HAV is then adsorbed or coprecipitated with aluminum hydroxide to provide adjuvant and carrier effects.

Because the dosage required for immunological efficacy of this product is very low, it is convenient to complex or adsorb the inactivated HAV onto a carrier. Aluminum hydroxide has been found to be quite acceptable for this purpose as it forms a tight complex with the HAV and prevents loss of the virus onto the walls of the container.

Accordingly, this invention discloses a commercial scale process for the preparation of hepatitis A virus of greater than 95% HAV protein purity, which comprises the steps of:

a) culturing hepatitis A virus in a large quantity in cell sheets, in large surface area static surface reactors,

b) removing the hepatitis A virus from the cell culture, by detergent permeation of the cell sheets such that the hepatitis A virus is liberated from the cell sheet without substantial removal, lysis or disruption of the cell sheet culture,

c) optionally removing non-hepatitis A virus specific nucleic acids at this stage from the harvested HAV, either by nuclease treatment or ion exchange adsorption of the nucleic acid,

d) concentrating the hepatitis A virus removed from the cell culture, using membranes and diafiltration, or capture by ion exchange,

e) removing residual non-hepatitis A virus specific proteins from the concentrated hepatitis A virus of step (d), by a combination of organic extraction, PEG precipitation, ion exchange, including removal of contaminating nucleic acid if not previously completed in step c), followed by gel permeation chromatography as a final polishing step, and

f) recovering the purified hepatitis A virus from step (e).

In a preferred embodiment of this invention, the process for commercial scale production of an inactivated hepatitis A virus vaccine comprises the steps of:

(a) Culturing a large quantity of Hepatitis A virus (HAV) in NUNC CELL FACTORIES, COSTAR CUBES, or even larger surface area static surface bioreactors in which a cell sheet of MRC-5 cells has been established on a regular array of solid stainless steel, titanium, or titanium stainless steel mesh;

(b) Harvesting the cultured HAV by removal of the culture medium and addition of a harvest solution containing about 0.05 to 0.5% Triton X-100, preferably 0.1%;

(c) Treating the harvested HAV with a nuclease to digest contaminating cellular nucleic acids;

(d) Concentrating the nuclease treated HAV by capture on an ion exchange column, and eluting the captured HAV with about 0.35 M NaCl, followed by precipitation with polyethylene glycol;

(e) Extracting the concentrated HAV with chloroform/isoamyl alcohol (24:1,v/v) with vigorous agitation, and separating and retaining the aqueous phase;

(f) Chromatographing the aqueous phase from the organic extraction on an ion exchange matrix at about 0.12 M NaCl, and eluting the HAV with a gradient to a higher NaCl concentration (1 M is adequate) and immediately diluting the pool of eluted HAV to a salt concentration of about 0.15M or below and preferably an HAV concentration of about 20 μg/ml or below;

(g) Chromatographing the HAV eluted from ion exchange on a size exclusion chromatographic resin, preferably a tandem arrangement of TOYOPEARL HW55S and HW65S in which the HAV enters the included volume of the matrix;

(h) Inactivating the gel filtered HAV by treatment with formalin at 1/1000, followed by sterile filtration, co-

precipitation with aluminum hydroxide, and dispensation into unit doses equivalent to about 25 ng -100ng per dose of inactivated purified HAV.

The HAV obtained in this invention is highly purified and is highly antigenic. The product is a purified preparation of hepatitis A virus wherein greater than 95% of the protein is hepatitis A virus specific based on SDS PAGE and silver stain analysis, and less than 0.1% of the mass of the preparation is non-HAV nucleic acid and less than 4% is lipid. The level of carbohydrate detectable as glucose in a hydrolyzed sample of the product is between about 0% and 200% of the mass of the protein present, with non-glucose carbohydrate being present at less than 15% of the protein mass. In preferred embodiments, the glucose and non-glucose carbohydrate, such as ribose, is present at less than about 20% of the protein mass. The preparation retains its anitgenicity when it is formalin inactivated.

Small quantities of the inactivated virus are adequate to elicit protective immune responses. Preferably, a dose of 25 -100 ng is administered once, twice or three times over a period of several months, as needed to boost high titers of anti-HAV immune responses.

The purified preparation of hepatitis A virus has the following characteristics, with each characteristic reported on a per μg of protein basis. The method in parenthesis was used for determination of the characteristic:

| | |
|---|---|
| a) lipid content (gas chromatography): | <0.1μg; |
| b) carbohydrate detected as glucose: (TFA hydrolysis, Dionex) | <0.1μg-2.5μg; |
| c) non-glucose carbohydrate: (TFA hydrolysis, Dionex) | <0.2μg; |
| d) hepatitis A virus specific protein (amino acid analysis and western blot), | >0.95μg; |
| e) hepatitis A virus specific RNA | 0.1-0.2μg |
| f) contaminating DNA | <0.0004μg. |

The presence of a carbohydrate component consisting primarily of glucose in the product is considered insignificant in view of the tolerability, immunogenicity, and protective efficacy of the inactivated adjuvanted vaccine. However, it is significant that levels of non-glucose carbohydrate (probably ribose from HAV specific RNA degradation) are very low in the product. It is also significant to note that it has only become possible to define the characteristics reported here since the development of a process capable of producing large enough quantities of the product to permit the relevant assays to be run.

In one specific formulation of this invention, the hepatitis A virus vaccine has the following nominal composition per 25 -100 unit/0.1-1 mL dose:

| COMPONENT | AMOUNT |
|---|---|
| Sodium Chloride | 900-9000 μg |
| Aluminum | 50-500 μg |
| sodium tetraborate | 3.8-38 μg |
| Formaldehyde | < 0.4 μg |
| Glucose (polymer) | 0-1000 ng |
| Protein | 25-100 ng |
| Chloroform | <44 ng |

EP 0 583 142 A2

| HAV RNA | 2-20 ng |
|---|---|
| Non-glucose carbo. | 0-20 ng |
| Fatty acid | <20 ng |
| Neomycin | <4 ng |
| MRC-5 DNA | <0.03 ng |
| Bovine serum albumin | <0.1 ng |
| BENZONASE | <0.0001 ng |

In another specific formulation, the hepatitis A virus vaccine has the following nominal composition per 25 unit/0.5 mL dose:

| COMPONENT | AMOUNT |
|---|---|
| Sodium Chloride | 4,500 µg |
| Aluminum | 250 µg |
| sodium tetraborate | 19 µg |
| Formaldehyde | < 200 ng |
| Glucose (polymer) | <20 ng |
| Protein | 25 ng |
| Chloroform | <22 ng |
| HAV RNA | 5 ng |
| Non-glucose carbo. | 4 ng |
| Fatty acid | <1 ng |
| Neomycin | <0.5 ng |
| MRC-5 DNA | <0.005 ng |
| Bovine serum albumin | <0.02 ng |
| BENZONASE | <0.00002 ng |

The purified inactivated hepatitis A virus of this invention has been found to be efficacious in preventing infection when a 25 ng (equivalent to a 25 unit dose of one of the above formulations) sample, based on EIA analysis relative to a reference standard that was characterized by amino acid analysis, is administered. No serious adverse experiences are observed among healthy adults, adolescents or children which could be considered to be vaccine related. Doses ranging from 6 to 50 units have been administered to adults, adolescents and children. Faster, higher seroconversion rates in adults have been observed as the doses administered are increased. This phenomenon is less pronounced in children.

In one month after a single 25 unit dose, 83% of adults ($\geq$ 18 years) and 97% of children and adolescents (2 to 17 years) seroconvert. Two doses of 25 units given two weeks apart increases the seroconversion rate in adults to 93%. Seroconversion seven months after a second or third 25 unit dose results in greater than 99% seroconversion in both age groups. Persistence in seropositivity is about 100% up to12 months and 36 months in both age groups. Thus, it is clear that a single 25 unit dose is sufficient to achieve seroconversion in at least 80% of any recipient population within one month of immunization. Immunization with a larger dose induces seroconversion in a higher percentage of recipients.

Based on a 25 ng dosage, a single COSTAR CUBE can be harvested according to the process of this invention to yield between about 5,000 and 10,000 doses or more. The process is also amenable to harvest and processing of several COSTAR CUBES at the same time. A single SSR with or without gauze to increase sur-

12

face area, results in significantly greater numbers of doses being generated per production run.

The following examples are provided to exemplify specific embodiments of this invention, but the examples should not be construed as the only mode of carrying out the various steps of this invention.

EXAMPLE 1

HEPATITIS A VIRUS STOCK SEED MANUFACTURE

A large-scale procedure for virus seed production involves the infection of MRC-5 cell monolayers in 6000 cm² NUNC CELL FACTORIES. MRC-5 cells are grown in the factories to confluent monolayers, and then infected with virus at an MOI of about 0.1. Following infection the cells are incubated for 28 days with weekly replacement of medium containing 10% v/v fetal calf serum. It has been found that high concentrations of serum, 2 to 10% v/v allow greater virus production than low levels, 0.5 to 2% v/v. At the end of this cycle the supernatant fluid contains large amounts of infectious virus, in this example $10^{7.3}$ $TCID_{50}$ per milliliter, which is harvested directly from the NCF, without cell lysis, and used as the source of virus stock seed. In this manner, the large quantities of infectious virus necessary for manufacture are obtained by a large scale method more reproducible and facile than roller bottles or flasks, or with mechanical harvest of the cells.

EXAMPLE 2

SCALE-UP OF VACCINE PRODUCTION

To support Phase III clinical studies, the scale-up of the laboratory process became an important consideration for the manufacture of Hepatitis A vaccine. To facilitate scale-up to the manufacturing process, modifications to previously known propagation and purification processes for HAV were required. Two major methods of cell propagation, NUNC CELL FACTORIES and Static Surface Reactors, such as the COSTAR CUBE, were used. This example describes the NUNC CELL FACTORY Process. Example 3 describes the COSTAR CUBE SSR Process.

Nunc Cell Factories used for the Monroe Efficacy Study

A revised procedure was developed which allows for improved scale-up of the process for generating larger amounts of attenuated Hepatitis A virus than has previously been possible. This was initially accomplished by replacing standard roller bottles with NUNC CELL FACTORIES (NCF). The basic unit of a NUNC CELL FACTORY (NCF) is a tray with a culture area of 600 cm². The trays forming the NCF are manufactured from polystyrene, treated for optimum cell attachment and spreading (Maroudas, 1976, J. Cell Physiol. Vol. 90. pp. 511-520). The 10 tray unit described here has 10 identical culture units (trays) with a total surface area of 6,000 cm². In two comers there are specially designed openings forming vertical tubes which interconnect trays in multiple layers. These vertical tubes terminate in two adapters accepting air filters and a specially designed Teflon connector. All units are sterilized by irradiation and are supplied by the manufacturer ready for use.

The MRC-5 cell environment in the NCF is very similar to that obtained in roller bottles. The cells are grown in static culture with the cells adhered to a solid substratum covered with a liquid medium and the supply of nutrients and oxygen to the cells is via diffusion. Use of NCFs enhances the reproducibility of operations while mimicking conditions used in roller bottle production. However, the method of harvest and down-stream processing is different, as further described below.

EXAMPLE 3

The COSTAR CUBE Static Surface Reactor used for Commercial Scale Manufacturing Process

The further scale-up challenge was to obtain as much surface area as possible per reactor while allowing good environmental control throughout the bioreactor. The NUNC CELL FACTORIES provided a partial solution to this challenge. Better solutions were provided by the COSTAR CUBE and STATIC MIXER Static Surface Reactors (SSRs). The former consists of densely packed polystyrene plastic sheets in a sterilizable plastic cube (COSTAR CS2000). A total of 85,000 cm² of usable growth surface is available in a single cube (compared to 6,000 cm² for a 10 tray NUNC CELL FACTORY). In the latter reactor, described in U.S, Patents 4,296,204 and 4,415,670, metal, ceramic, glass, or plastic static mixing elements form a densely-packed growth surface; these elements can be fabricated and arranged to provide higher surface areas than the COSTAR CUBE in a single reactor for large manufacturing processes. HAV has been successfully cultured in both types of reactor. The SSR has a circulation loop which permits medium recirculation and replenishment of the nutrients through an aerator in order to control the pH and dissolved gas levels in the medium, to meet the oxygen demand of the culture, and to achieve medium perfusion. This optimization is not possible in roller bottles or in the NCF. In addition, a medium change was made in order to improve availability of large quantities of a key raw material. Iron supplemented calf serum (Cat. A-2151, Hyclone Laboratories, Inc., Logan, UT or equivalent) has been used successfully to replace fetal bovine serum.

EXAMPLE 4

Harvest of Hepatitis A Virus from NUNC CELL FACTORIES or Static Surface Reactors by Triton Lysis

Neither the NUNC CELL FACTORY nor the Static Surface Reactor is amenable to mechanical scraping for release of MRC-5 infected cell sheet. The cell-associated Hepatitis A virus was efficiently recovered using Triton cell lysis, and purified using a novel process. The solution obtained with the Triton lysis procedure was more dilute than the preparation obtained from mechanical scraping of roller bottles. The Triton harvest was concentrated by diafiltration, followed by treatment with the absorbent, XAD, to remove residual Triton. Clearance of Triton to less than 10 $\mu$g/mL has been demonstrated using a Capillary Zone Electrophoresis in-process assay. The HAV was then precipitated with polyethylene glycol (PEG), extracted with chloroform:isoamyl alcohol. Contaminating DNA was removed using a DNA filter column, followed by anion exchange and size exclusion chromatography. The process involving both NCF propagation and the Triton lysis did not adversely affect the immunogenicity of the purified formalin-inactivated Hepatitis A vaccine.

EXAMPLE 5

Nuclease Treatment and Capture Chromatography

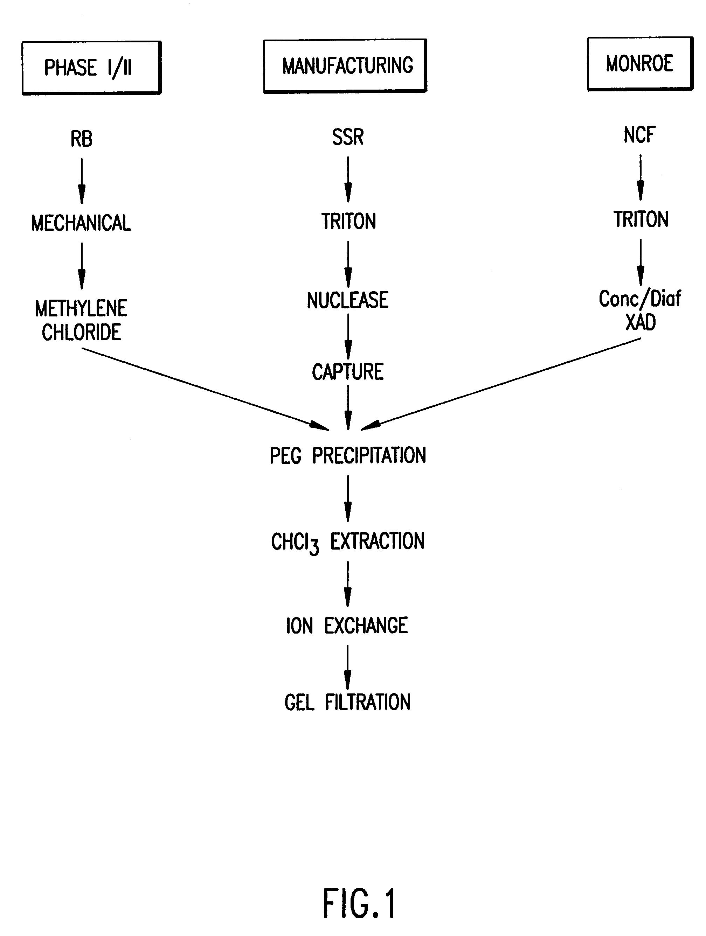

The scalability, productivity and consistency of the process was enhanced by treatment of the Triton harvest with an endonuclease followed by capture on anion exchange chromatography to concentrate virus (see Figure 1). This modification eliminated the need for a concentration/diafiltration step, the XAD treatment and the DNA filter column.

A nonspecific endonuclease was selected which is active against both RNA and DNA. This endonuclease, BENZONASE, is isolated from Serratia marcescens and cloned in Escherichia coli. It is produced as a very highly purified, well characterized recombinant protein by Nycomed Pharma A/S (Denmark). This particular endonuclease was selected, in part, because it has very high specific activity and can be added directly to the crude Triton harvest in very small quantities (10 mcg BENZONASE/L harvest). Test methods utilizing a WESTERN Blot for BENZONASE specific protein and a kinetic assay for enzyme activity were developed to measure removal of the enzyme by the purification process. Most (>90%) of the added endonuclease activity can be accounted for in the wash and flowthrough fractions of the anion exchange chromatography capture step (described below). The BENZONASE level is reduced by a factor of more than 100 in the capture column product.

EXAMPLE 6

ADDITIONAL PROCESS STEPS

a) Capture Step
The Hepatitis A virus is concentrated from the nuclease treated Triton harvest on an anion exchange column by loading at low ionic strength and step eluting in 0.35 - 0.5M sodium chloride in phosphate buffer. The Hepatitis A virus is concentrated 30-fold by this capture step and Triton is reduced to undetectable levels as determined by the in-process CZE assay.
b) PEG Precipitation and Chloroform Extraction
The Hepatitis A virus from concentration/diafiltration/XAD or that eluted from the capture column is precipitated with polyethylene glcol (PEG). Under proper conditions this step is highly selective and a major contaminant, which otherwise copurifies with the virus, remains soluble and is discarded in the supernatant fraction. The PEG precipitate containing virus is resuspended and then extracted with chloroform:isoamyl alcohol (24:1) to remove soluble lipids and proteins denatured by the chloroform which remain at the interface between the aqueous and organic phases.
c) Anion Exchange Chromatography and Size Exclusion Chromatography

14

The aqueous extract of the chloroform extraction step is loaded onto an anion exchange column at low salt and subsequently eluted with a NaCl gradient to 1.0 M salt. A final size exclusion chromatography step yields a highly purified virus preparation (see Figure 2).

d) Inactivation

The formalin inactivation of Hepatitis A virus has previously been carried out using 93 $\mu$g formaldehyde/ml (1:4000 formalin) and a temperature of 37°C, for antigen concentrations near dosage levels. Under these conditions the inactivation of virus followed first order decay kinetics with a half-life of approximately 2 hours, which corresponded to a loss of about 3.5 $\log_{10}$ $TCID_{50}$ (50% tissue culture infective dose) per day. A reduction of 7 logs of virus infectivity was achieved in about 2 days, but the time of inactivation was extended to twenty days to ensure a large margin of safety that the virus had been completely inactivated.

Small scale experiments confirmed that increasing the formaldehyde concentration increases the rate of inactivation as measured by loss of $TCID_{50}$. The rate of inactivation varied proportionally with the formaldehyde concentration, as expected for pseudo-first order kinetics of inactivation. Tissue culture testing on two hundred dose equivalents confirmed complete inactivation when the virus was incubated with 370 $\mu$g/mL formaldehyde (1:1000 formalin) virus at 37°C for 5 days. Thus, these modified conditions of inactivation provided the same margin of safety as those previously used. This has been confirmed with three large pilot-scale inactivations carried out under identical conditions, followed by aluminum hydroxide adsorption to provide vaccine for clinical testing. The rate of inactivation was about four times the rate previously observed at lower formaldehyde concentrations, in excellent agreement with small scale studies. Samples of these two aluminum hydroxide adsorbed vaccines were tested for immunogenicity in mice, with $ED_{50}$ for each similar to that of previous vaccines (less than 2 ng).

e) Aluminum Hydroxide Adsorption

Adsorption onto aluminum hydroxide has been carried out by coprecipitating formalin inactivated virus with aluminum hydroxide. The method used for all clinical preparations has been essentially the same, with the only variation being the concentration of virus in the formalin inactivated bulk solution. Direct precipitation of inactivated virus onto aluminum hydroxide was carried out by first adding potassium aluminum sulfate to the formalin inactivated bulk solution. Finally, the precipitate was formed by adding sodium hydroxide. Formaldehyde and residual salts were eliminated by removing supernate from the suspension and replacing it with physiological saline.

## EXAMPLE 7

## PURITY OF THE HAV PRODUCT

Analytical size exclusion chromatography (HPSEC), EIA and protein as well as SDS-PAGE are used for the purification process monitoring to ensure consistent yields of high purity vaccine. The HPSEC provides a means to monitor the loss of nucleic acid during nuclease treatment as well as an assessment of purity in the latter steps of process. The chromatograms in Figure 2 show that the peak corresponding to the Hepatitis A is apparent in the PEG precipitate. The Hepatitis A virus is enriched in the solvent extraction step and then concentrated in the ion exchange step. The final gel filtration yields a highly purified (>95% by SDS-PAGE and silver staining) Hepatitis A preparation. As an independent measure of purity the HAV product is a single included symmetric peak in the HPSEC assay (see figures 2, 37 and 38).

## II. Clearance of Impurities

CZE analysis of in-process samples indicates that the Triton is removed by the concentration diafiltration step in the Monroe process and by the capture column in the manufacturing process. In addition, the BENZONASE flows through the capture column. The amount of BENZONASE present at the anion exchange product stage is below the level of detection (<18 femtograms BENZONASE activity and less than 13pg BENZONASE mass/25 unit dose Hepatitis A; the limit of detection in this assay is 12.5 pg).

## EXAMPLE 8

## Purification of Hepatitis A virus harvested from NUNC CELL FACTORIES or Static Surface Bioreactors by Triton Lysis: Monroe Study

The HAV was propagated essentially as described in EXAMPLE 2 using NCFs. MRC-5 cells were grown in batches of sixty 6000 $cm^2$ NCF's at 37°C for 7 days using a medium comprised of William's Medium E supplemented with 10% v/v fetal calf serum and Neomycin sulfate. At day 7, the cells were refed with fresh medium containing sufficient virus to infect the cultures at about 0.1 MOI, and transferred to a 32°C environment. The cultures were further incubated at 32°C for 21 days with weekly refeeding. The HAV was harvested by treating the cell sheet with a lysing buffer containing 0.1% Triton X-100, 10 mM TRIS, 1mM $MgCl_2$ pH 7.5. The resulting lysate from the NCF's was pooled and frozen pending further processing.

Four liters of frozen Hepatitis A virus harvested from NUNC CELL FACTORY culture by treatment with TRITON X-100 were thawed in a 20-30°C water bath for 4 hours and then filtered. The filtered lysate was concentrated 10-fold on a Pellicon apparatus using a 300,000 molecular weight cut off membrane. The concentrated lysate, approximately 400 mL, was subsequently diafiltered against 10 mM Tris-HCl buffer, pH 7.5 containing 150 mM NaCl and 1 mM EDTA. Triton was removed by batch treatment with AMBERLITE XAD-4 (Rohm and Haas Company) polymeric resin at 30 mg/ml of lysate. Following filtration to remove the XAD-4, the lysate was adjusted to 510 mM NaCl and then precipitated by the addition of polyethylene glycol (PEG 8000, Sigma) to 5% (v/v) final concentration. The mixture was vigorously shaken, incubated at 4°C for 1 hour and then centrifuged at 1000 x g at 4°C for 10 minutes. Following centrifugation the supernatant fluid was removed and discarded.

The precipitate was then dissolved in 6.2 mM sodium phosphate buffer, pH 7.2 containing 120 mM NaCl and 1 mM EDTA. The resuspended pellet was then extracted with an equal volume of a mixture of chloroform:isoamyl alcohol (24:1, v/v). The mixture was shaken vigorously and then centrifuged at 3000 x g for 10 minutes at 20°C. The interface containing insoluble material was reextracted with the same organic solvent mixture using 30% of the initial volume. Following centrifugation, the two aqueous phases were combined. The chloroform extract was then adjusted to 350 mM NaCl and chromatographed on a DEAE-Sepharose CL6B (Pharmacia) in a filtration mode to adsorb DNA. The column flowthrough fraction containing HAV was collected and diluted by a factor of three with 6.2 mM sodium phosphate buffer, pH 7.5 and chromatographed on a TOYOPEARL DEAE 650M (Toso Haas) with a gradient elution to 1 M NaCl. The Hepatitis A eluted at 15-30% of 1 M buffer corresponding to 15-30 mS. Fractions pooled from this region were subsequently chromatographed on Sepharose CL4B (Pharmacia) in 6.2 mM sodium phosphate buffer with 120 mM NaCl. The load for this column was designed to be greater than 1% but less than 5% of the column volume. The product eluted between 46 and 64% of the total column volume.

The final purified bulk was 0.22 micron sterile filtered and inactivated with 1:4000 formalin reagent (formalin is 37-40% by weight formaldehyde) at 37°C for 20 days. These conditions exhibited first order decay kinetics with a half-life of 2 hours and a loss of approximately 3.5 $log_{10}$ $TCID_{50}$/24 hours. The inactivation period was extended to 20 days to assure a large margin of safety. Potassium aluminum sulfate was added to the inactivated Hepatitis A and then the solution was precipitated by the addition of sodium hydroxide to pH 6.6-6.8. Formaldehyde and residual salts were removed by centrifugation in which the supernatant fluid was removed and replaced with physiological saline.

## EXAMPLE 9

Purification of Hepatitis A, harvested from COSTAR CUBES with Triton X-100, using Nuclease Treatment and Capture Chromatography: Production Scale Process

Production scale material has been prepared using the SSR process for virus propagation. In this process, the COSTAR CUBE was used as the growth surface for the cells; the CUBE was connected to a COSTAR CS2000 aerator using an external loop and pump to drive medium flow, as described in Example 3. MRC-5 cells were inoculated into a COSTAR CUBE SSR, and grown at 37°C for 7 days. Perfusion was started two days after cell introduction. The reactor was continuously perfused with William's Medium E supplemented with 10% v/v iron-supplemented calf serum and Neomycin sulfate. Reactor pH was controlled between 6.9 and 7.6, and dissolved oxygen in the reactor ranged between 15 and 100% of air saturation in the medium. Cell growth was allowed to proceed for a total of 7 days, and virus was introduced into the reactor at approximately 0.1 MOI, using virus stock prepared according to the method of Example 1. The SSR temperature was reduced to 32°C, and feeding continued for 21 days. The SSR was then drained of medium and refilled with a 0.1% w/v TRITON®, 10 mM TRIS®, pH 7.5 buffer to release the HAV. For four lots of virus produced by this method in COSTAR CUBE systems as shown in Example 10, HAVAg production averaged 2.8 times the historical average in NCF reactors using the Monroe propagation process, on a surface area basis. The increased SSR productivity allowed facile scaleup of the propagation process to manufacturing scale.

The crude lysate is treated with BENZONASE to release virus from nucleic acid adducts followed by capture of the virus on anion exchange chromatography. The BENZONASE treatment eliminated the need for the concentration/diafiltration step, the XAD-4 treatment and the DNA filter column (See Example 8).

Twenty-five liters of COSTAR CUBE Triton X-100 harvest were treated with 5 -25µg BENZONASE/L lysate in the presence of 1 mM MgCl2 and then filtered through a 0.22 micron filter. The mixture was incubated for 18 hours at 25°C. The enzyme-treated lysate was loaded on a TOYOPEARL DEAE 650M anion exchange column equilibrated with 4.7 mM sodium phosphate buffer, pH 7.2 containing 90 mM NaCl. The Hepatitis A was eluted (approximately 800 ml) with 6.2 mM sodium phosphate buffer containing 0.35 M NaCl and 1 mM EDTA. Test methods utilizing a kinetic assay for enzyme activity were developed to monitor the removal of the enzyme in the process. Results indicate that >90% of endonuclease activity can be accounted for in the capture column wash and flowthrough fractions. Moreover, the total nuclease activity, including that of the added BENZONASE, in the capture column product was reduced by a factor of >100 as compared with the amount initially present in the lysate.

The NaCl concentration of the Hepatitis A product from the capture column was adjusted to 420 mM and the virus was precipitated with polyethylene glycol (PEG 8000, Sigma) at a final concentration of 4.5%. The mixture was stirred vigorously and then incubated for 1 hour at 4°C. Following incubation, the precipitate was collected by centrifugation at 1000 x g for 10 minutes at 4°C. The supernatant fluid was removed and discarded. The PEG precipitate was resuspended in 6.2 mM sodium phosphate buffer, pH 7.2 containing 120 mM NaCl and 1 mM EDTA. Organic extraction of the resuspended PEG precipitate was accomplished by the addition of 1.5 volumes of chloroform:isoamyl alcohol (24:1, v/v). Phase separation was enhanced by centrifugation at 3000 x g for 10 minutes at 20°C and the upper, aqueous phase was retained. The interface was reextracted with 30% of the original volume of organic mixture, centrifuged and the second aqueous phase was combined with the first.

The aqueous extract was chromatographed on a TOYOPEARL DEAE 650M (Toso Haas) anion exchange matrix and eluted with a gradient to 1 M NaCl. The Hepatitis A virus eluted at 15-30% of the 1M gradient corresponding to 15-30 mS. Fractions were pooled from this region and subjected to size exclusion chromatography. The size exclusion step utilized two columns, TOYOPEARL HW55S and HW65S, in tandem. Under these conditions the Hepatitis A virus was in the included volume for both matrices and the tandem column arrangement was used to resolve both higher and lower molecular weight impurities from the Hepatitis A virus. The columns were equilibrated with 6.2 mM sodium phosphate buffer, pH 7.2, containing 120 mM NaCl. The Hepatitis A eluted in a symmetrical peak and product fractions were pooled.

The 0.22 micron sterile filtered product from the size exclusion chromatography was inactivated at approximately 500 units/mL (one unit corresponds to about 1 ng of viral protein) with 370 µg/mL (1:1000 dilution of formalin which is 37-40% by weight formaldehyde) for 5 days at 37°C. The inactivated Hepatitis A virus was 0.22 micron sterile filtered. Coprecipitation with aluminum hydroxide was carried out by the initial addition of potassium aluminum sulfate and then titration to pH 6.6-6.8 with sodium hydroxide. Residual salts and formaldehyde were removed by settle/decant method in which the supernatant fluid was removed and replaced by physiological saline.

EXAMPLE 10

The product produced by the process, as described above, has been analyzed for: protein by amino acid analysis (gas phase hydrolysis followed by PITC labeling and reversed-phase high performance liquid chromatography); HAV antigen by HAV antigen solid-phase enzyme immunoassay (antigen capture using a human polyclonal antibody preparation, followed by detection with a mouse monoclonal antibody and goat-anti-mouse antibody/peroxidase conjugate); carbohydrate (including glucose and non-glucose carbohydrate detection; non-glucose carbohydrate was present in the product at a level of less than about 0.1 µg per µg of product protein) by high pH anion exchange chromatography and pulsed amperometric detection (HPAEC-PAD, acid hydrolysis using 2 N TFA followed by HPLC analysis); host cell DNA by DNA hybridization (dot-blot procedure using an Alu DNA fragment as the hybridization probe); fatty acid by gas chromatography (methyl esterification of the fatty acids followed by capillary gas chromatography); RNA by hydrolysis and reversed-phase HPLC analysis of ribonucleotides.

In addition to the data presented below, generated according to the foregoing methods, SDS-PAGE analysis and protein detection by silver stain or by western blot with anti-HAV antibodies revealed only the presence of HAV specific proteins upon loading 50-300 ng of protein. In addition, the presence of full HAV capsids was demonstrated by analysis of HAV specific RNA by hybridization with HAV specific DNA probes, sucrose density gradients and reversed phase HPLC. HAV specific RNA was found to be present in the product at about 0.1 - 0.2 µg per µg of protein. This level of HAV specific RNA is consistent with a ratio of about 1/3 full capsids to about 2/3 empty HAV capsids. Finally, an independent measure of HAV purity is provided by the assay shown in Figure 2, using HPLC analysis, which shows a single HAV product peak.

The absolute concentrations of protein, carbohydrate, lipid, and DNA are presented in section A below. The section A data are transformed in section B below by normalization to micrograms of protein, and in section C, as a weight percent of protein (meaning the ratio of the mass of carbohydrate present, for example, and the mass of protein present, multiplied by 100).

The results of these analyses are shown below for eight preparations of HAV prepared according to the commercial scale process of this invention.

A. Composition Analysis

| Lot No.. | Protein (mcg/mL) | RNA (mcg/mL) | Total Carbohyd (mcg/mL) | DNA[*] (mcg/mL) | Fatty Acid (mcg/mL) |
|---|---|---|---|---|---|
| 1 | 14.7 | 2.4 | 3.4 | <0.0045 | <0.3 |
| 2 | 8.0 | 1.1 | 3.7 | <0.0003 | <0.3 |
| 3 | 18.9 | 2.1 | 0.9 | <0.0003 | <0.3 |
| 4 | 17.0 | 1.5 | 2.5 | <0.0003 | <0.3 |
| 5 | 16.6 | 2.5 | 2.2 | <0.0003 | <0.3 |
| 6 | 8.4 | 1.5 | 1.1 | <0.0003 | <0.3 |
| 7 | 14.5 | 1.4 | 1.5 | <0.0003 | <0.3 |
| 8 | 14.6 | 1.8 | 1.2 | <0.0003 | <0.3 |

| Lot No.. | RNA | Weight % protein Carbohyd | DNA | Fatty Acid |
|---|---|---|---|---|
| 1 | 16 | 23 | <0.036 | <2.0 |
| 2 | 14 | 46 | <0.004 | <3.8 |
| 3 | 11 | 5 | <0.002 | <1.6 |
| 4 | 9 | 15 | <0.002 | <1.8 |
| 5 | 15 | 13 | <0.002 | <1.8 |
| 6 | 18 | 13 | <0.004 | <3.6 |
| 7 | 10 | 10 | <0.002 | <2.1 |
| 8 | 12 | 8 | <0.002 | <2.1 |

*The limit of detection (LOD) for the first sample was 0.0045 µg/mL. For subsequent samples, the sensitivity was increased such that the LOD was 0.003 µg/mL.

The foregoing data characterize the product of this invention, prior to inactivation and aluminum hydroxide co-precipitation, as an HAV preparation having the following characteristics, on a per microgram of protein basis (with protein being greater than 95% pure HAV protein by SDS PAGE and silver staining):

| | |
|---|---|
| Carbohydrate composed of glucose | 0.1 - 2.5 µg |
| Non-glucose carbohydrate | < 0.2 µg |
| DNA | < 0.004 µg |
| Lipid | < 0.1 µg |
| HAV RNA | 0.1 - 0.2 µg |

Preferably, these analyses are performed on the purified HAV product of this invention, when the HAV product is most concentrated, prior to inactivation and formulation. However, provided with sufficient quantities of inactivated or finally formulated vaccine, these analyses would predictably yield substantially similar results. In one particularly preferred embodiment, the vaccine of this invention has the following nominal composition per 25 unit/0.5 mL dose:

| COMPONENT | AMOUNT |
|---|---|
| Sodium Chloride | 4,500 µg |
| Aluminum | 250 µg |
| sodium tetraborate | 19 µg |
| Formaldehyde | < 200 ng# |
| Glucose (polymer) | <20 ng*# |
| Protein | 25 ng*# |
| Chloroform | <22 ng*# |
| HAV RNA | 5 ng*# |
| Non-glucose carbo. | <4 ng*# |
| Fatty acid | <1 ng*# |
| Neomycin | <1 ng*# |
| MRC-5 DNA | <0.005 ng* |
| Bovine serum albumin | <0.02 ng*# |
| BENZONASE | <0.00002 ng*#& |

* Extrapolated from analyses of purified virus bulk.

# From analysis of demonstration lot samples.

& Based on activity assay.

EXAMPLE 11

Comparison of Different HAV Preparations

The purified HAV product of this invention was compared with the description of other HAV products as described by their manufacturers. The results of this comparison are shown in Table 1:

## TABLE 1

## COMPARISON OF INACTIVATED
## HEPATITIS A VACCINES

| Vaccine | Total Protein (ng/dose) | Virus Protein (ng/dose) | Protein Purity |
|---|---|---|---|
| This invention | 25[b]-100 | 25[c]-100 | >95[e,f] |
| SmithKline Beecham | 2000[b] | 300-500[d] | ≤25[f] |
| Japan[a] | ----- | 1000[d] | >95[e] |

a.   Joint effort by Chemo-Sero-Therapeutic Research Institute, Chiba Serum Institute, and Denka Bio-Research Institute.

b.   Based on amino acid analysis (AAA) data.

c.   Defined by HAV antigen enzyme immunoassay; calibration standard was purified virus, purity >90% by SDS-PAGE/silver stain, protein defined by AAA.

d.   Defined by HAV antigen enzyme immunoassay; calibration standard undefined.

e.   Defined by SDS-PAGE analysis.

f.   Defined by HAV antigen to total protein ratio.

From this data and the information presented above in Example 10, it is apparent that the product of this invention is more pure (based on HAV antigen to total protein ratio and amino acid analysis), more completely characterized, and contains the lowest amount of virus protein effective to achieve seroconversion and protection than any product previously reported for which analytical data is available.

EXAMPLE 12

Vaccine Efficacy - Monroe Study

Vaccine was prepared by infection of MRC-5 cell sheets cultured in NUNC CELL FACTORIES with CR326F HAV. The culture was maintained for several weeks by regular feeding. At the appropriate time, infected MRC-5 monolayers were harvested by detergent treatment and the HAV was purified according to the process of this invention shown in figure 1 for NCF's. Aqueous virus was adsorbed to aluminum hydroxide and stored at 2-8°C. Doses of 0.6 mL each contained 25 units of hepatitis A virus antigen based on radioimmunoassay against a purified virus reference standard, and 300 μg of aluminum hydroxide. Placebo, contained 300 μg of aluminum hydroxide and thimerosal at a 1:20,000 dilution. Healthy, seronegative vaccine recipients at high risk of contracting HAV infection received either a single 0.6 mL dose of placebo or vaccine. Blood samples were taken on the day of vaccination and one month later. Almost 100% of the vaccine recipients were free of HAV infection after a 50 day waiting period was taken into account to screen out individuals that had contracted HAV infection prior to vaccination. On the other hand, a significant number of placebo recipients contracted HAV infections after the 50 day screening period. The study population was sufficiently large so that the protection of vaccine recipients is statistically significant.

## EXAMPLE 13

### Virus Production In Static Mixer Reactor

Hepatitis A virus was propagated in a SSR consisting of a static mixer column connected to an aerator by an external loop and pump system (Charles River Biological Systems CRBS 5300). The static mixer reactor was fabricated of titanium metal elements totalling about 260,000 $cm^2$ surface area, in a column 8" diameter by 40" long. MRC-5 cells were introduced into the reactor and grown for 7 days at 37°C in William's Medium E supplemented with 10% v/v fetal calf serum and Neomycin sulfate. Cell growth was allowed to proceed for 7 days, and a portion of the medium was exchanged thrice weekly, to give a feeding rate equal to the NCF Monroe process. Virus was introduced into the reactor at approximately 0.1 MOI. The SSR temperature was reduced to 32°C, and feeding continued as above for 22 days. The SSR was then drained of medium and refilled with a 0.1% w/v Triton® buffer to release the HAV. This reactor produced approximately 50% more HAVAg on a surface area basis than a T-flask control inoculated using the same reagents, and fed, propagated and harvested in a similar manner. The reactor's HAV production also compared favorably with the Nunc cell factories of Example 2.

## EXAMPLE 14

### HAV Production On Stainless Steel Gauze Static Mixer Elements

### 14.1 MATERIALS AND METHODS

#### Cell culture.

MRC-5 cells, at PDL $\leqq$ 40, were used in all studies. Cells were cultured in Williams E Medium containing 10% iron enriched calf serum, 2 mM glutamine, and 50 mg/L neomycin sulfate at 37° C. 0.1% Pluronic F-68 was added to medium in studies employing a sparged stirred tank reactor. A cell density of 10,000 cells/$cm^2$ was used to inoculate new culture surfaces. Cell counts were performed using a Coulter Counter and/or hemacytometer with Trypan blue staining to test viability.

#### Cell culture on metal coupons.

A solid or gauze metal coupon, ca. 5 cm x 5 cm, was placed in a glass, 150 $cm^2$ petri dish and sterilized. After allowing to cool, 90 mL of medium was placed in the dish, and the cell inoculum was then added. The medium amount allowed for complete coverage of the metal surface. After 1 day at 37° C the metal piece was removed using sterile forceps, and placed in a new sterile petri dish (to discount cell growth on the glass surface) containing 90 mL of medium. The solid sheets were trypsinized by washing twice with phosphate-buffered saline (PBS), and adding 2 mL of trypsin to the metal. After observing the release of cells, the trypsin was pipetted gently over the surface several times to ensure complete removal.

#### Virus stock and infection.

Hepatitis A virus stock, CR326F P28, with a titer of 108 $TCID_{50}$/mL, was used in all studies. MOI values reported here are calculated as $TCID_{50}$/cell and not PFU/cell. Cultures were infected by adding the virus stock to fresh medium and adding this to the culture. After infection cultures were incubate at 32° C.

#### Viability Staining on Gauze Coupons.

Cell viability was monitored by the use of fluorescein diacetate (FDA) and ethidium bromide (EB) staining. In viable cells FDA is cleaved by esterases in the cytoplasm to yield free fluorescein that fluoresces green. EB rapidly intercalates into the nuclear DNA of non-viable cells and fluoresces red. Cells were labelled with both dyes simultaneously. The staining solution consisted of 100 μL of FDA stock (5 mg/mL in acetone) and 200 μL of EB stock (10 mg/mL in PBS) in 50 mL of PBS. The gauze material was then incubated in 5 mL of this solution for 5-6 min. After labelling, the support was washed twice in PBS (20 mL per wash) and taped between a slide and coverslip. The samples were then viewed using a BioRad MRC-600 confocal microscope.

Static mixer reactor.

Gauze (known as "E-pack") and solid sheet 316 stainless steel static mixer elements (SMV configuration) were obtained from Koch Engineering (Wichita, KS). All elements were approximately 2"×2", constructed of type 316 stainless steel or titanium. The surface area of the solid elements was estimated to be 1300 cm$^2$ and the gauze elements were estimated to be 2300 cm$^2$. The surface area to liquid volume ratio of these latter elements was approximately 26 cm$^2$/mL (ca. 24 cm$^2$/mL based on total reactor volume). Five elements were normally placed in a threaded 5 cm x 26.5 cm glass columns with PTFE end fittings. Elements were placed in a configuration with the mixing directions perpendicular in successive elements. The column with elements comprises the reactor for cell growth. Stirred tank satellite vessels coupled to electronic controllers were used to control the medium pH at 7.2 - 7.3 and the DO at 80-90%. Medium was recirculated from the stirred tank to the reactor by use of a peristaltic pump.

Reactor planting was accomplished by providing a low circulation rate to allow cell attachment. Inoculum was placed into the system and typically recirculated at 100 mL/min for 5 min to mix the cells and medium. Very little cell attachment occurred during this rapid recirculation period. The flow rate was then reduced to ca. 2 mL/min, which provided a superficial velocity through the reactor approximately equal to the sedimentation velocity of the cells. Velocities around the cell sedimentation rate were found to be optimal for cell attachment rates and even distribution of cells through the reactor.

Cell lysis procedure.

Cells were lysed in a Triton lysis buffer which contained 0.1% Triton X-100, 10 mM TRIS, and 1 mM MgCl$_2$. T-flasks were washed twice with 0.3 mL/cm$^2$ PBS, and lysed twice with 0.1 mL/cm$^2$ lysis buffer for 1 hr. Protocols for lysing reactors is described in the text.

Analytical Methods

Cellular metabolites were measured using a Kodak Ektachem DT60 analyzer. Hepatitis A Viral Antigen (HAVAg) was assayed using a monoclonal antibody based enzyme immunoassay. Total protein of cell lysates was quantified using a Coomassie Blue Bio-Rad protein assay (Cat. #500-0001) against a bovine serum albumin standard.

RESULTS AND DISCUSSION

Growth of MRC-5 cells on Solid Metal Coupons.

Static mixer work prior to this study was performed on titanium elements. It was desireable to investigate cell growth on surfaces of different alloys that, in addition to being less expensive than titanium, provide greater ductility for ease of static mixer manufacturing. Two grades of each stainless steel and Hastelloy were investigated: 316 and 316L stainless steel, and Hastelloy grades B and C. Metal coupons were inoculated with approximately 10,000 cells/cm2. After 4 days, the cells were trypsinized and the final cell densities recorded:

| Material | Cells/cm$^2$ |
| --- | --- |
| Titanium | $1.4 \times 10^5$ |
| 316 Stainless | $1.6 \times 10^5$ |
| 316L Stainless | $1.2 \times 10^5$ |
| Hastelloy B | $2.2 \times 10^3$ |
| Hastelloy C | $1.3 \times 10^4$ |

Growth on both grades of stainless steel was similar to that on titanium; cells did not grow on hastelloy B and grew poorly on hastelloy C. Differences in cell densities noted for the stainless steels and titanium should not be construed as evidence that one is superior; differences are likely due to assay variability.

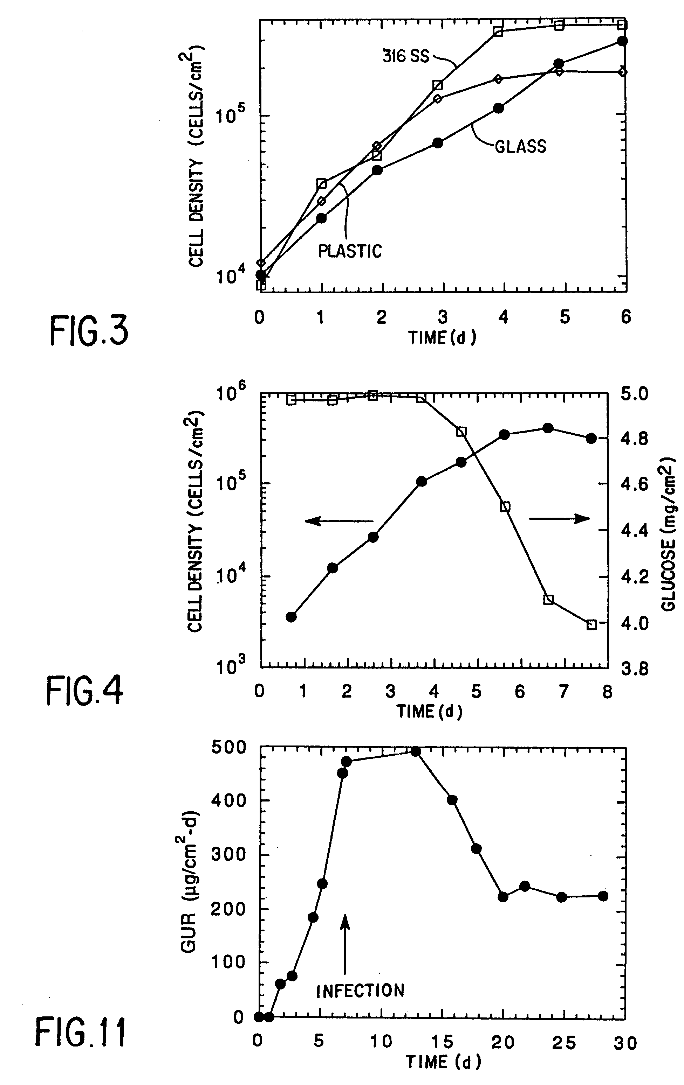

Most of the data for Hepatitis A virus processes have been obtained for cells grown on plastic supports, e.g., polystyrene tissue culture flasks. For comparison, growth curves of MRC-5 cells on polystyrene, glass, and 316 stainless are shown in Figure 3. The cells grow comparably on all of the surfaces. The maximum specific growth rates are similar (0.034 h$^{-1}$ for plastic, 0.032 h$^{-1}$ for glass, and 0.039 h$^{-1}$ for 316 stainless steel). The lower final cell density obtained for plastic is likely due to nutrient limitation. Growth on plastic was performed in T-flasks while growth on glass and stainless steel was performed in petri dishes. Due to the difference in the volume of medium used per cm2 in each sytem, an imbalance in available nutrients results. Cell growth and the consumption of glucose for stainless steel coupons are shown in Figure 4.

Growth of MRC-5 on Gauze Metal Coupons

In an effort to maximize surface area per unit volume, metal gauze material was investigated in petri dishes for MRC-5 cell growth. For certain combinations of wire diameter and spacing, a piece of gauze with the same projected area as a solid coupon can present a greater overall surface area for cell growth. Sheets of gauze can then be formed or arranged into a reactor in such a way as to maximize the available surface area per unit of reactor volume. To test growth of cells ona gauze surface, MRC-5 cultures were conducted by the techniques described in the Materials and Methods section.

Growth of MRC-5 cells was demonstrated on gauze constructed of either 316 stainless steel or titanium. Gauze coupons with wire diameters ranging from 100 μm to 400 μm, and weaves of 14 x 100, 120 x 110, 40 x 200, and 107 x 59 wires per inch were investigated. Cells grew on all samples, to greater or lesser extents. In the early stages, cells grow on the wire surface. Shortly thereafter cells begin to bridge the interstices, especially at the corners. With time the cells completely fill the interstices and form multilayers. By visual inspection, the gauze supports very high cell densities that are more than scaled by surface area. Direct cell surface concentrations are not available since multilayer growth on the gauze is not amenable to trypsinization. Preliminary data indicated that interstice size is an important consideration, as cells are unable to bridge large interstices, and gauze with small interstices reduces in the limit to the same surface as a smooth plate. Material of construction did not affect cell growth, and it is concluded that any biocompatible material which can be woven into gauze sheets is amenable to use. The metallic gauzes used possess the advantage of being easily cleaned and reused for many cycles of cell growth, as described below.

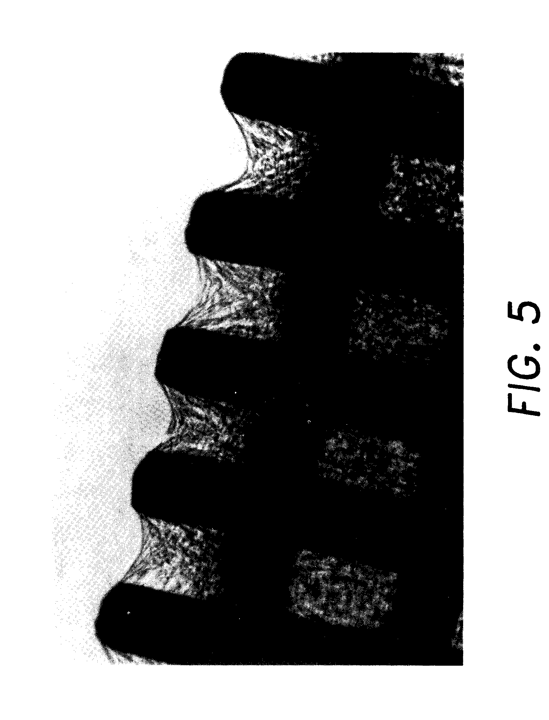

From this point on only 316 stainless steel gauze with a weave of 107 wires x 59 wires per inch, and a wire diameter of 160 μm (resulting in a wire spacing of 77 μm x 270 μm) will be cited. The gauze static mixer elements discussed below are constructed of this material. In addition to providing increased surface area, the gauze allowed cells to grow in multilayers. A micrograph of multilayered cells grown on this gauze is shown in Figure 5. It was of interest to determine if the cells throughout the layers are all viable or if the cells in the center of the multilayer are necrotic. Fluorescein diacetate and ethidium bromide were used for this purpose as detailed in the Materials and Methods section. By scanning cross-sections of the gauze using a laser confocal microscope, it was evident that the vast majority of the cells were viable based on the facts that ethidium bromide did not intercalate the nuclear DNA and that fluorescein diacetate was cleaved to yield free fluorescein. Figure 6A shows the fluorescein signal for a series of cross-sections through the cell layers. Figure 6B shows the ethidium bromide signal for the same sections. Separate coupons of cells made non-viable by treatment with methanol served as a positive control for the ethidium bromide and a negative control for fluorescein diacetate in these experiments. This control is shown in Figure 7.

Planting cells on gauze static mixer elements.

A uniform plant is important for optimal culture performance and for maintaining a low, uniform cell doubling level for biologicals manufacturing. Gauze static mixer elements composed of the same material investigated above were obtained. The cylindrical reactor was oriented with the axis vertical. Although early studies were conducted with the reactor axis horizontal to promote cell sedimentation onto the growth surface, it was found that attachment to a vertical surface is as efficient, and is the preferred mode of operation. Throughout this work cells were planted at approximately 10,000 cm2. Cells have been planted in multiple element reactores where the mixing axes of the individual elements are anywhere between parallel or ninety degrees to each other. For purposes of fluid mixing and optimal reactor operation, the perpendicular configuration is preferred.

Cells did not plant in early studies employing a recirculation rate which gave 5 cm/min superficial velocity. Planting efficiency was increased by filling the reactor and allowing the cells to plant by gravity sedimentation for 2 hr. This routinely provided a planting efficiency of greater than 90% (planting efficiency = number of cells planted/total number of cells in inoculum x 100%), however the plant was nonuniform. As shown in Figure 8, the bottom element contained the most cells while the top element contained the fewest. From these data, an average settling velocity of the MRC-5 cell preparation was calculated to be 6 cm/h. In a further experiment using four elements, medium was recirculated for planting at a rate approximately equal to this settling velocity.

A uniform plant was reproducibly obtained with a planting efficiency of greater than 90% for this condition (Figure 8). The above results demonstrated that medium superficial velocity through the reactor influences both the rate of attachment of the cells and the ultimate distribution of cells in the reactor.

A comparison of cell growth on solid sheet and gauze static mixer elements.

Two reactors were set up and run simultaneously to delineate the differences between solid sheet and gauze elements. The solid elements were constructed of titanium, and the gauze elements were constructed of stainless steel. The surface areas were estimated to be 1300 $cm^2$ for the solid elements and 2300 $cm^2$ for the gauze, resulting in a gauze to solid surface area ratio of 1.8. The solid sheet elements operating in conjunction with the gauze elements provided the control for all experiments. Reactors were planted with approximately 10,000 - 20,000 cells/$cm^2$. Reactors were run in a batch mode until the glucose concentration fell below 120 mg/dL, at which time medium perfusion was started. Both reactors were inoculated with the same harvest of MRC-5 cells, and were fed from a common reservoir of medium to eliminate any differences in these variables.

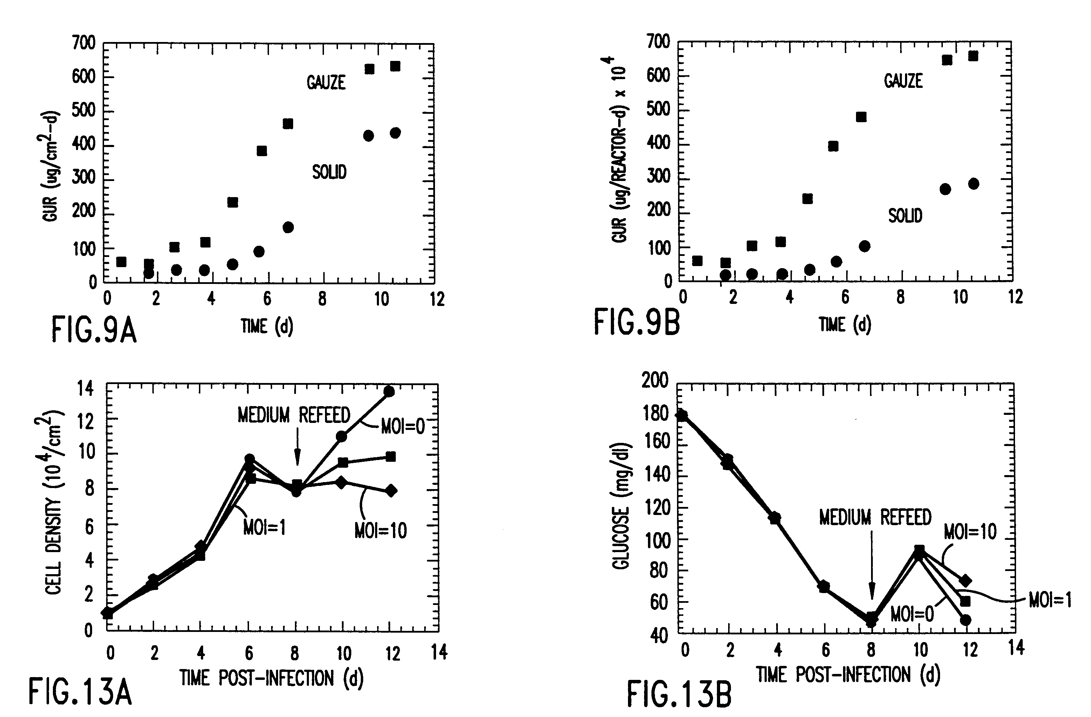

The glucose uptake rate (GUR) for each reactor is shown in Figure 9. Figure 9A compares the reactors on a $cm^2$ using the areas referred to above; these surface areas are considered approximate. Figure 9B compares the systems on a reactor medium volume basis since each reactor was the same size, i.e., five 2"x2" elements with a 1 L satellite medium vessel. Figure 9B does not embody any approximations regarding surface area. Glucose uptake rate is employed as a measure of cell growth and was used to estimate the onset of stationary phase. The last point in Figure 9 indicates the time the reactors were harvested by triton lysis. Cells were visually confluent on the solid matrix and interstices on the mesh were almost filled on the gauze elements, suggesting that more growth was possible. At this point, the medium was perfused at a rate of 0.19 L/h for the solid reactor, and the glucose concentration in the medium at this point was 1.16 g/L. The perfusion rate was 0.26 L/h for the gauze reactor, and the medium glucose concentration was 0.69 g/L. These results demonstrate the superior metabolic activity that is obtained for the gauze reactor versus the solid reactor for the cell growth phase.

The reactors were drained and the cells were lysed using a detergent buffer to extract cellular protein, a measure of the cell mass in the reactor. For these experiments, the lysis procedure to harvest the cell-associated protein from each reactor was identical: 2 L of room temperature PBS was circulated through the reactors at 100 mL/min for 5 minutes and then drained. This wash to remove medium proteins was repeated twice. Next, 1 L of the lysis buffer in the Materials and Methods section was recirculated through the reactor at 400 - 500 mL/min, 37° C, for 1 hr. This was repeated once (lysates 1 and 2), and subsequent lysates circulated longer - 2 hr for lysate 3, and 4 hr for lysate 4. The percentage of protein removed in each lysate is presented in Figure 10. When total protein calculations are converted to a cell density by the correlations presented in Appendix 1, the solid static mixer elements contained 3x$10^5$ cells/$cm^2$ while the gauze elements contained 6x$10^5$ cells/$cm^2$. The former number is believed to be representative for the solid sheet reactor since all of the cells were removed during lysis as demonstrated by swabbing the elements and viewing the swab under the microscope. However, all of the cell debris was not removed from the gauze elements as observed by microscopy, so the latter number is predictably low. On a reactor basis, the gauze elements supported greater than 3x the cell concentration as the solid elements. Provided surface area calculations are accurate, this difference in total protein is in excess of the ratio of the surface areas and is evidence of multilayer cell growth.

Production of HAVAg on gauze static mixer elements.

HAVAg production was demonstrated for a reactor employing gauze static mixer elements. The objective of this experiment was to determine if viral propagation occurred on stainless steel gauze elements supporting multilayer growth; it was not an optimized system for the production of HAVAg. For this run, complete medium replacements were employed rather than medium perfusion. A GUR profile for this run is shown in Figure 11; the GUR's calculated for this figure are based on glucose values obtained the day after medium was refed. Cells were infected with an MOI of ca. 1 on day 7. Cell density for MOI calculations was estimated based on the glucose consumption rate at the time of infection. As observed with processes such as Example 9, the glucose uptake rate plateaus shortly after infection and is followed by a decline, consistent with cell infection. The observed decline in Figure 10 is more precipitous than that observed with the process of Example 9, and was likely due to a more uniform degree of infection (Example 9 employed an MOI of 0.1). The viral antigen was harvested 21 days after infection by Triton detergent lysis. The protocol for lysing the cells is outlined below.

Lysate 1: recirculate lysis buffer through static mixer for 1 hr at 37° C.

Lysate 2: recirculate lysis buffer through static mixer for 1 hr at 37° C.

Lysate 3: sonicate for 1 hr in Branson sonicator bath containing 0.7 L lysis buffer.

Lysate 4: freeze elements in 1 L lysis buffer for 12 hr, thaw, and sonicate for 1hr.

The percent of total protein and HAVAg released in these washes is indicated in Figure 12. The first 2 lysates employing recirculation were very effecient in removing HAVAg from the cells. This experiment employed only 2 PBS washes before lysis, and was not completely efficient, therefore the total protein percent of lysate 1 is artificially high and that of 2, 3, and 4 are low. A single pooled sample was assayed for HAVAg by EIA prior to freezing. Based on the percentages of each lysate determined later, this correlated to an antigen yield per surface area twice that of Example 9. The objective of this experiment was realized in that viral propagation as indicated by HAVAg yield occurred. This experiment demonstrates the utility of the gauze static mixer reactor for production of HAV.

Although the reactor was likely harvested late based on the GUR profile, the antigen yeilds were relatively high, indicating susceptibility of multilayers to infection. The data also indicate the importance of MOI in HAV production; by infecting at a MOI of 1, it appears that the process duration should have been decreased by more than one week and harvested on day 20. Based on a variety of data, the time of infection and MOI are important variables for a manufacturing process; these parameters are important in decreasing the length of the culture. Cell growth experiments indicate that infected cells continue to grow at a rate similar to uninfected cells, even for cultures infected at high MOI with CR326F P28 (Figure 13). This is despite infection and a temperature to 32° C, the growth permissive temperature for this strain of virus. The maximum specific growth rate calculated from these curves is 0.015 h$^{-1}$, approximately half the growth rate of uninfected cells at 37° C. The fact that the cells behave similarly during the first week of HAV infection is important in the operation of a reactor, in that one would like to realize the highest cell concentration possible regardless of infection time and MOI. This experiment demonstrates that optimization of cell growth duration, infection duration, and MOI are intertwined.

Cleaning the static mixer elements.

It is desirable to possess a reusable growth surface for biologicals manufacture to eliminate waste disposal, enable automation of the reactor process, and enhance culture reproducibility. Currently, the gauze elements are rinsing with distilled water to remove medium salts (500 mL/min for 1 h), placed in 1 L of a 5% (w/v) NaOH solution and autoclaved for 45 min. After cooling, elements are rinsed with distilled water until free of NaOH (as determined by rinsate pH). This procedure is extremely effective in removing cell debris by microscopic inspection. It is speculated that a much less harsh treatment would result in equivalent cleaning, and be more appropriate for a clean-in-place manufacturing reactor. Gauze elements treated by this process have been reused for at least ten cycles with no change in cell growth properties. Elements in the above virus growth study were prepared in this manner, demonstrating the utility of used, cleaned elements for HAV propagation.

CONCLUSIONS

These studies have demonstrated that static mixer elements constructed of stainless steel gauze are able to support multilayer growth of MRC-5 cells. Multilayers were demonstrated to be viable, with no indication of necrosis. Infection studies indicate that these multilayers are susceptible to hepatitis A infection and in a non-optimized system produce antigen in excess of what is currently realized using solid supports such as in Example 9. With microcarrier culture, cell clumping may result in ill-defined multilayers; the gauze statix mixer provides well defined multilayers due to the crafted geometry, and therefore nutrient limitation and waste accumulation pose less of a problem. Cell growth within the interstices of the mesh closes off the interstices, and creates a plug flow pattern similar to that of solid sheet elements. Therefore, the system reaps the mixing benefits of static mixer technology and leads to predictable and uniform nutrition to the cells. This is in contrast to other packed bed reactor types such as randomly packed beds of fibers, packed beds of glass beads, hollow fiber reactors, or ceramic monolith reactors. In these types of reactors, cell growth causes deterioration of the medium flow patterns and creates poorly nourished pockets of cells. In summary, a reusable reactor containing stainless steel gauze static mixer elements embodies great potential for the cultivation of MRC-5 cells and the production of hepatitis A antigen.

EXAMPLE 14 - APPENDIX 1

SUMMARY

Total protein from MRC-5 cell lysates was quantified and compared with direct cell counts (by hemacytometer and the use of a Coulter Counter). Healthy cells in the early exponential, late exponential, and stationary growth phases as well as cells at 1, 2, and 3 weeks post infection demonstrated that total protein measurements from each culture lysate could be directly related to cell density by use of a single plot. This simple correlation provides a means to estimate the cell density in reactors that are not amenable to direct counting techniques.

DISCUSSION

Protein assay and sample preparation.

The Bio-Rad protein assay (Cat. #500-0001) is a rapid, sensitive assay used to quantify total protein. Early experiments demonstrated that the current lysis buffer (0.1% Triton X-100, 10 mM Tris-HCl, 0.1 mM MgCl2) does not interfere with the Coomassie Blue based assay. For consistency all dilutions, including those of the standard protein (bovine serum albumin), are made in triton lysis buffer. The fact that triton cell lysates are often flocculent in nature indicates that samples may require further treatment prior to being assayed. Large flocs lead to high protein titers. However, after two cycles of rapid freeze (in -70° C ethanol) and thaw, the floc size was reduced to the degree that a homogeneous suspension was obtained. Furthermore, by assaying several dilutions of a single sample, extraneous points that may result from a poorly prepared sample are easily detected. Samples were also centrifuged and the clarified supernatant used; this treatment provided similar results but was deemed unnecessary. Data presented here are for cell lysates frozen a minimum of two times and well vortexed prior to being assayed.

Protein concentration as a function of cell density.

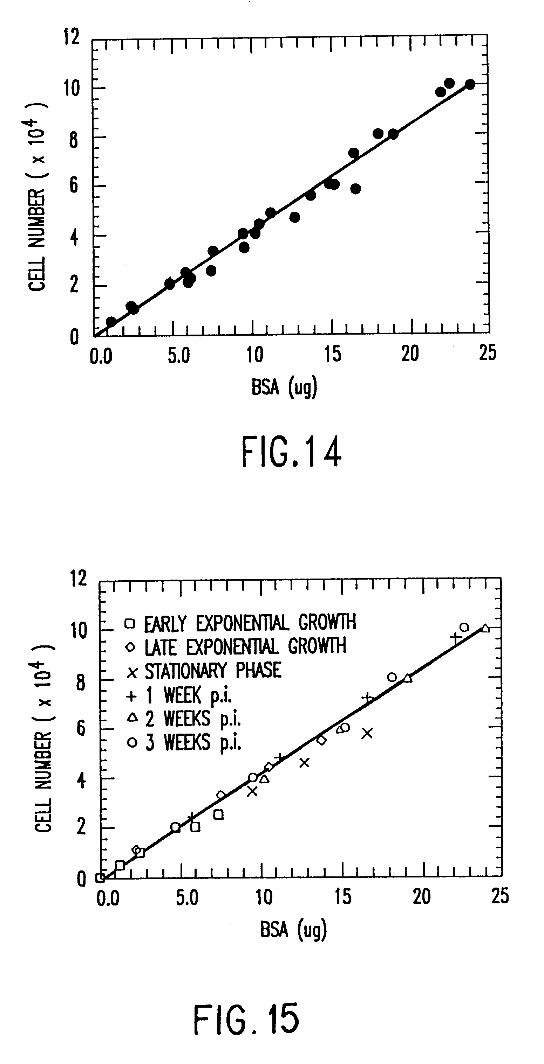

For a single standard curve to allow conversion of total protein to a specific cell number, cells throughout the process must be comprised of an approximately equal quantity of protein. To test this, T-flasks were inoculated with an equal number of cells. At specified intervals in the process, two flasks were sacrificed. One flask was trypsinized and cells counted by hemacytometer and a Coulter Counter while the other flask was subjected to detergent lysis, i.e., washed twice with 0.3 mL/cm$^2$ PBS, lysed twice with 0.08 mL/cm$^2$ lysis buffer. This was done for cultures in the early exponential growth phase, late exponential growth phase, and stationary phase, as well as for cultures at 1, 2, and 3 weeks post infection. The cumulative data for these experiments is presented in Figure 14. The best fit line is described by the equation: $Y(10^4) = -0.09 + 0.42X$. This figure demonstrates that a single curve provides a means to estimate cell number by total protein measurements, regardless of the "state" of the cells.

The cells in this experiment were infected with HAV CR326F P28 at an MOI of 1. At 3 weeks post infection, trypsinization proved ineffective in harvesting cells. However, since the cell densities from 1 and 2 weeks post infection were approximately equal, and visible cell lysis at 3 weeks did not appear to exist, cell densities used for 3 weeks were based on data obtained at 2 weeks. Figure 14 is replotted, indicating the time of each specific data point. By discounting the data at 3 weeks post infection, the slope and intercept of the curve is not greatly affected (see Figure 15).

Using the above correlation, total protein data for several culture lysates prepared as in Example 9 yielded an average of 490 μg/mL protein. This translates to approximately $3.3 \times 10^5$ cells/cm$^2$, and is in general agreement with cell densities determined using a hemacytometer on control T flasks. In summary, it is believed that total protein of Triton cell lysates provides a useful approximation of the cell density at the time of virus harvest.

EXAMPLE 15

Mixing Studies Of Solvent Extraction Step:

Summary

During purification of the Hepatitis A, the solvent extraction step was critical in removing impurities still present at the PEG pellet stage. In order to fully understand and control this step, a series of experiments was carried out to determine the important parameters for solvent extraction. Two key variables were identified: duration of mixing and intensity of shaking, as determined by the size of the bottles used. The ratio of solvent to aqueous volumes was not a significant factor over the range studied, neither was the use of a mechanical shaker as opposed to hand shaking or vortexing.

## Materials And Methods

Samples of resuspended PEG precipitate were mixed with a solution of chloroform and iso-amyl alcohol (24:1 $CHCl_3$:IAA) in various proportions from 0.5:1 to 3:1 (ratios of solvent to aqueous phases). Mixing was carried out by hand shaking vigorously, vortexing (for the 15 ml tubes) or on a 3D mechanical shaker (Glas-Col, Terre Haute, Ind) at a motor speed of 100 (with the tubes taped on horizontally for the 15 ml and 50 ml tubes). Mixing times ranged from 20 seconds to 1 hour.

After mixing, samples were centrifuged to separate the phases. For the 50 ml tubes and 500 ml bottles, a Beckman J6-MI centrifuged with a JS 4.2 swinging bucket rotor was used at 3800 rpm for 10 minutes. For the small tubes, initially a Dynac II centrifuge was used at 1000 rpm for 5 minutes, then for subsequent experiments, the samples were transferred to 50 ml tubes for centrifugation as above.

The aqueous phases were removed with a glass pipette and analyzed by sizing HPLC under the following conditions:two TSK P4000x1 columns (TosoHaas) in series, mobile phase was RCM 8 (also referred to as CM563, which is 150 mM NaCl in 6.2 mM phosphate buffer, pH 7.2) at 0.32 ml/min, monitored at 214 and 260 nm (80 min run time). Results are expressed as peak areas or ratios of peak areas based on 214 nm UV absorbance. For initial experiments in 15 ml tubes, a single TSK column was used (40 min run time). Subsequently, two columns were set up to obtain better resolution between the HEP A and impurity peaks.

Experiments were initially performed in 15 ml tubes on a 2-6 ml scale and samples were mixed by hand shaking or vortexing. When scattered results were obtained for the first few lots, mixing was carried out with larger volumes (in 50 ml tubes, filled to a maximum of 24 ml) on the mechanical shaker, Samples were also taken from the 500 ml bottles during processing of the manufacturing lots. The bottles were shaken for a short time (for example 20 sec). the phases were allowed to separate by sitting for about a minute and a 600 μl sample was removed from the upper phase. The bottle was returned to the shaker for another short time, then sampled again. These samples were later spun down in a micro centrifuge for 2 minutes at maximum speed.

## RESULTS

### 1. Mixing Time

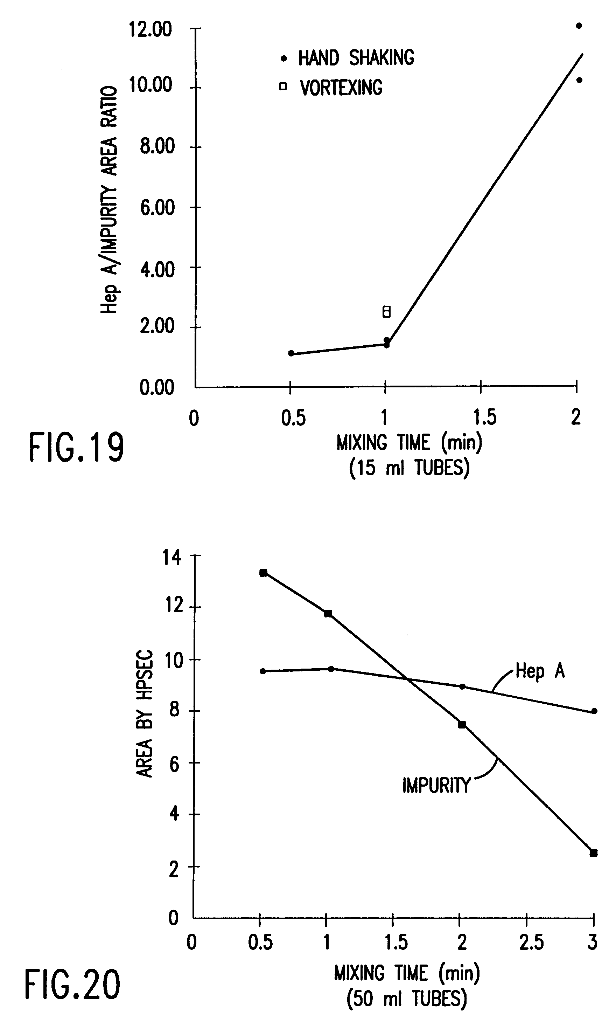

From preliminary small scale experiments in 15 ml tubes, mixing time appeared to be an important variable, with extended mixing time resulting in better removal of impurity, From figure 6, it is clear that the ratio of Hep A to impurity is increased by longer mixing times. In addition, in figure 20, we can see that the impurity peak area decreases significantly with mixing time.

With the 50 ml tubes, mixing time was shown conclusively to be important, with 3 minutes required in this example for highest removal of impurity (figure 20). In this case, analysis was carried out with the tandem columns for better resolution and only a slight decrease in Hep A area was observed.

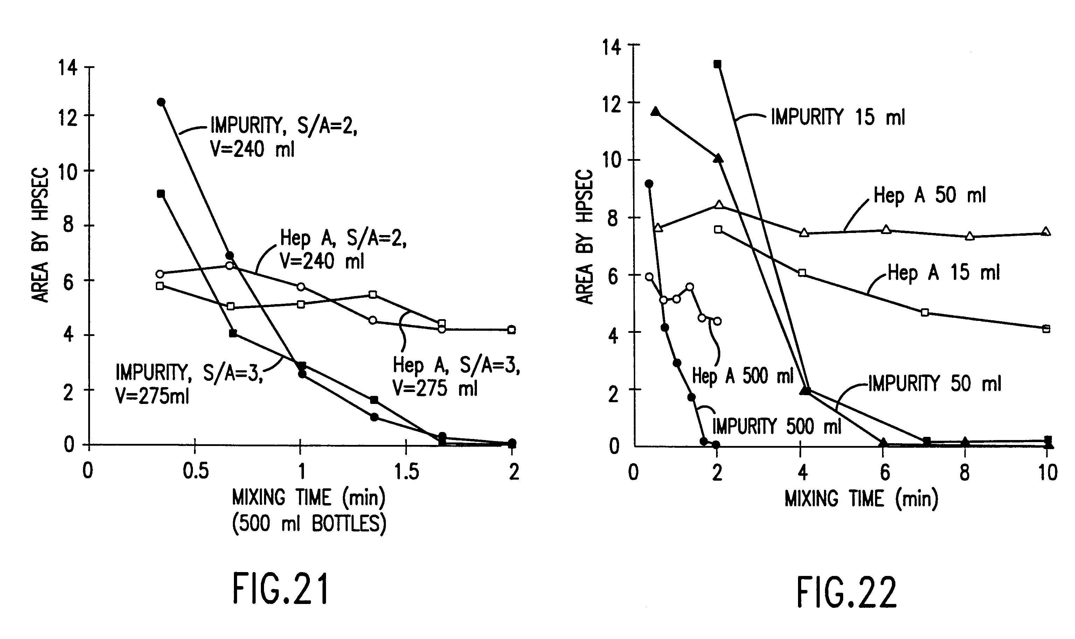

The effect of mixing time was also studied in the 500 ml bottles used in production. Samples were taken every 20 seconds over the course of 2 minutes total mixing time. This experiment was carried out at two different solvent ratios: 2:1 and 3:1, which also correspond to two different aqueous volumes in the bottles (about 60- ml and 90 ml, the solvent volume remained constant at 180 ml). Figure 21 shows that the level of impurity decreased with mixing time, being completely removed after 1 min 40 seconds. The time used in production has been 2 minutes, which ensures significant removal of impurity. It will be extended to 3 minutes for more complete removal. The differences in volumes and solvent ratios appear to have little effect over the range used.

This experiment was extended to longer mixing times in both 50 ml and 15 ml tubes. Figure 22 shows that the same degree of purity is eventually reached in all of the tubes used, but that it requires 6-8 minutes in the smaller tubes and less than 2 minutes in the 500 ml centrifuge bottles. These differences will be discussed further in section 3. In order to determine whether the virus was affected by prolonged shaking, a 50 ml tube was mixed for 1 hour on the mechanical shaker. HPSEC results showed no further decrease in Hep A peak area and EIA results (table 1) indicated that antigenicity is the same for 1 hour as for 2 minutes of mixing. The process stream results of the same lot show a similar EIA value. Therefore, in order to ensure complete removal of impurities in production, the minimum mixing time as seen on the graph can be extended with out reducing virus yield.

TABLE 1

| Effect of Prolonged Shaking on Hep A by HPSEC and EIA (50 ml tubes) | | |
| --- | --- | --- |
| Duration of mixing | Hep A/Impurity ratio | EIA (U/ml) |
| 2 min | 0.74 | 23,800 |
| 1 hour | ∞ (impurity not detectable) | 24,500 |
| Process stream (2 min, 500 ml bottles) | 8.6 | 20,000±16% |

## 2. Volume Ratio

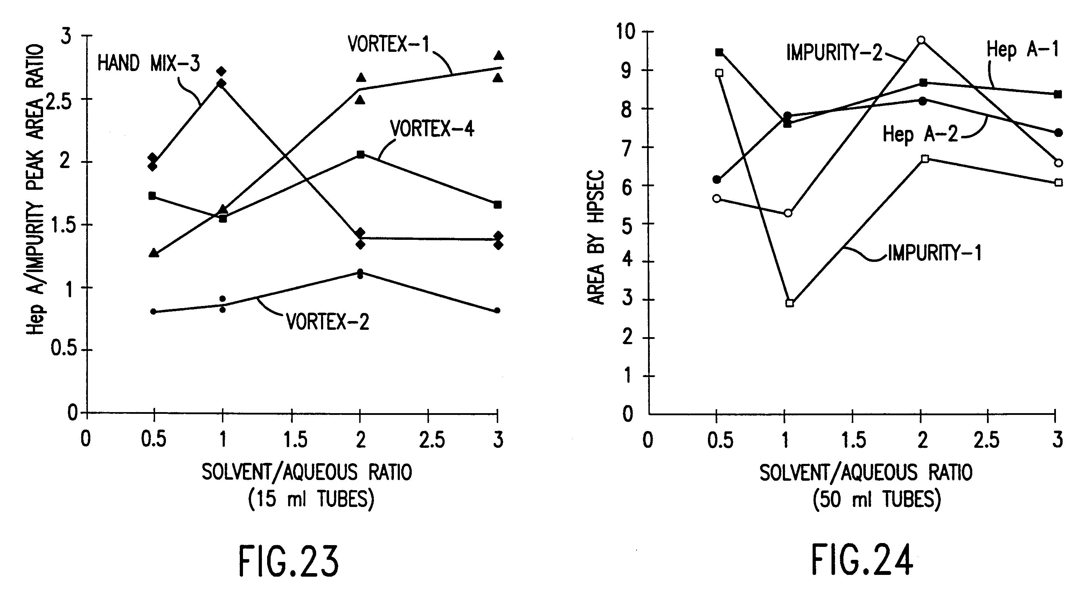

The volume ratio of solvent to aqueous phases for a single extraction was 1.5 for the pilot lots (stirred in a single bottle with a magnetic stir bar), and between 2 and 3 for the demonstration lots (shaken in 500 ml bottles). The effect of a volume ratio between 0.5 and 3 on purity was therefore examined. Initial small scale experiments (in 15 ml tubes, mixed for 1 min) were performed with four different production batches of starting PEG precipitate. Figure 23 showed that no clear correlation was observed between the solvent to aqueous ratio and the removal of impurity. The scatter in the data is probably due to variability in the starting material and inconsistency of shaking. The sample size was scaled up to 50 ml tubes and the mechanical shaker was used for 2 minutes with the tubes taped on horizontally. Results are shown in Figure 24. Again, although there is scatter in the data, there is no clear trend based on solvent to aqueous ratio. This was confirmed by mixing time experiments in the 500 ml bottles with 2:1 and 3:1 solvent ratio (figure 21) showing very little difference in impurity removal. It appears therefore that solvent ratio is not an important variable, and the increase in solvent ratio did not contribute to the improved purity seen in the consistency lots.

## 3. Type Of Mixing And Size Of Vessel

No trend could be seen for the effect of hand shaking vs. vortexing in the small (15 ml) tubes since the results were scattered for experiments from different production lots (figure 23). The single data point on figure 19 also shows good agreement between hand shaking and vortexing. In the larger tubes, hand shaking and mechanical shaking were compared. In figure 25, we can see that the hand shaking gives only slightly different results from the shaker for the same size and fill of tubes. Table 2 shows results form an experiment in 500 ml bottles, From a lot of six bottles, one was mixed by hand while the others were shaken mechanically. A sample was taken for each type of mixing and HPLC analysis showed that they were very similar, both giving high purity, confirming that the type of shaking is not critical but that the size of the bottle used in very important, and the 500 ml bottles are superior to 50 ml tubes for the same mixing time on the shaker. this is also illustrated by the data in figure 22.

TABLE 2

| Effects of Different Types of Mixing on Hep A/Impurity Ratio (2 min) | |
| --- | --- |
| Type of Mixing | Hep A/Impurity ratio |
| Mechanical shaking | 2.47 |
| Hand Shaking | 2.69 |

The level of fill of the tubes was also considered. The 50 ml tubes filled to a level of 10.5 ml (solvent + aqueous) gave very similar results to the typical fill of 21 ml. Similarly, 500 ml bottles filled with 240 or 275 ml showed equivalent amounts of impurity removal (figure 21). Over the course of all of the mixing experiments, 15 ml tubes were filled either to 0.13 or 0.4 of their full capacity. For 50 ml tubes, most were filled to 0.53 of capacity (with one experiment at 0.26), and 500 ml bottles were filled between 0.48 and 0.57 of full capacity. Since similar levels were used for the three types of tubes and the results obtained could not be correlated with the level of fill, it probably does not have a strong effect over this range. It is likely however, that if the tubes were completely filled, there would be no head space and it would be difficult to mix them. As a result, this would reduce the efficiency of impurity removal.

Another factor which varied and could be important is the width to height ratio of the tubes: 0.13 for the 15 ml tubes, 0.26 for the 50 ml tubes and 0.58 for the 500 ml bottles. It is possible that a larger width to height ratio is conducive to better mixing because the resulting shaking pattern gives rise to a larger interfacial area.

Data from earlier piloting lots with this same impurity present in the PEG pellet were also examined. Four lots had been solvent extracted for 1 minute in a single flask equipped with a magnetic stir bar or a spinner. The solution was then distributed into centrifuge bottles for phase separation by centrifugation. The solvent to aqueous ratio was 1.5. The resulting ratios of Hep A to impurity ranged from 0.48 to 2.7. An additional piloting lot was shaken in 250 ml conical centrifuge bottles for 1 minute with a solvent to aqueous ratio of 1. The resulting purity ratio (Hep A/impurity) was 0.87. These results are all comparable to those obtained in 15 or 50 ml tubes under similar conditions, but are much lower than the typical range of 4 to 21 (for 8 batches) obtained in the full-scale demonstration lots. This is consistent with the longer mixing time and larger bottles used for mixing at the production scale.

Conclusion

We can conclude from this data that the difference in purity between the full-scale demonstration lots and the earlier piloting lots is that shaking was carried out in larger (500 ml) bottles and for a longer time (2 min vs. 1 min). The volume effect is probably due to the larger interfacial area resulting from shaking in these bottles. The 500 ml bottles were typically filled to a level less than 300 ml, resulting in a large headspaced area which became filled during mixing to give rise to an extensive solvent/aqueous/air interfacial area. It is likely that a reduced interfacial area can account for the lower purity of the solvent extraction product obtained in small tubes. However, since mixing time is also an important variable, the purity in the 50 ml tubes eventually reaches the same level as in the 500 ml bottles.

The other variables that were investigated, solvent to aqueous ratio and type of shaking, were not found to be important over the range studied.

EXAMPLE 16

Solubility Of Hepatitis A Virus

As described previously, significant loss of Hepatitis A was observed based on HPSEC analysis of process streams from production lots. During purification it was estimated using HPSEC quantitation that more than 50% of the virus was lost overnight between the ion-exchange and size exclusion chromatography steps of the purification. Coincident with this loss was the appearance of a precipitate that was removed by centrifugation.

It was hypothesized that the precipitate was Hepatitis A, and that precipitation was driven by the higher ionic strength of the buffer used to elute virus from the ion-exchange column. Attempts to dissolve the precipitate were made with aqueous solution of sodium chloride and sodium phosphate. Based on the loss of virus using HPSEC quantitation the concentration of the precipitate (actually slurry of precipitate) was estimated at several mg/ml. Aliquots of the precipitate slurry were mixed with either 6 mM sodium phosphate, 0.15 N sodium chloride in 6 mM sodium phosphate, 0.3 N sodium chloride in 6 mM sodium phosphate, 0.5 N sodium chloride in 6 mM sodium phosphate, or 1 N sodium chloride in 6 mM sodium phosphate.

When mixed with even a small amount of 6 mM sodium phosphate the precipitate dissolved within a few seconds. However, when mixed with solutions containing sodium chloride, no great difference in appearance was observed (except obvious dilution). A stock solution of Hepatitis A was prepared by dissolving 0.35 ml of slurry with 5.65 ml of 6 mM sodium phosphate. It was noted that complete dissolution of visible particles took place after addition of only ca. 0.5 ml of the sodium phosphate solution. Samples of the stock solution were mixed with varying amounts of 1 N sodium chloride in 6 mM sodium phosphate to reach target concentrations of 0.15 N, 0.3 N, or 0.5 N sodium chloride (Table I).

TABLE 16-I

| Volume stock solution | Volume 1 N sodium chloride in sodium phosphate | Antigen dilution | sodium chloride molarity |
|---|---|---|---|
| 1.5 | .27 | 1.2 | .15 |
| 1.5 | .65 | 1.43 | .30 |
| 1 | 1 | 2 | .50 |

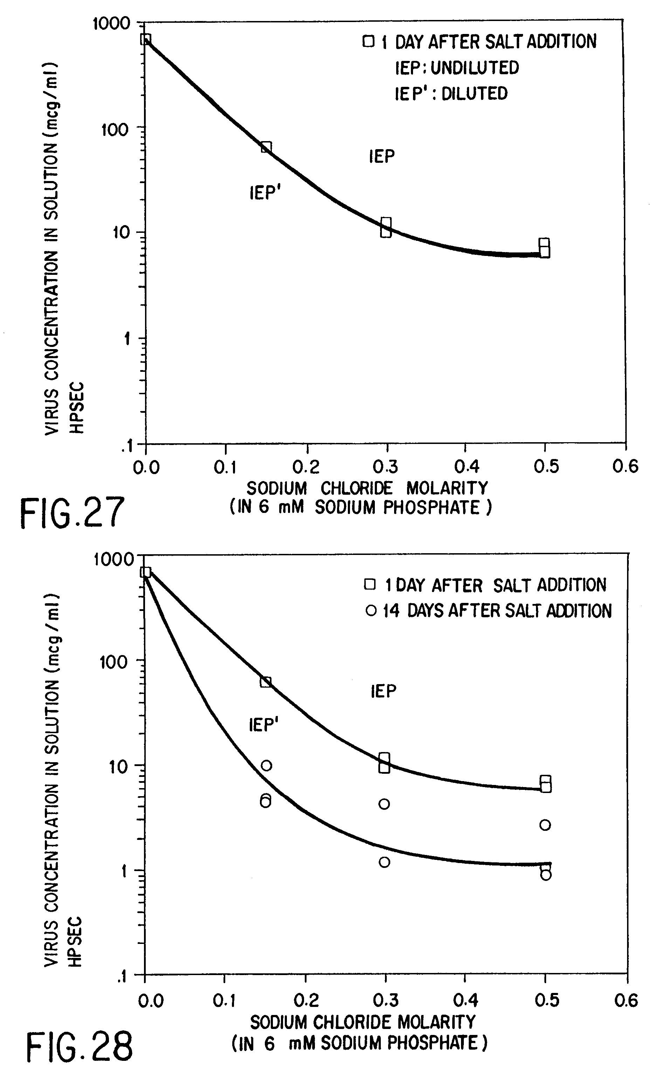

The concentrations of theses solutions were then quantitated using HPSEC, and then quantitated two weeks later by HPSEC. These data are presented in Table II and plotted in Figures 27 and 28.

Figure 27 shows the initial concentrations of Hepatitis A measured in solution approximately a day after the addition of salt. The stock solution was estimated to have a concentration of about 100 mcg/ml by HPSEC, which would correspond to about 2 mg/ml in the precipitated slurry. (The amount of Hepatitis A in this slurry then did roughly correspond to that loss observed during production. Since the precipitate was observed to dissolve with only a small amount of phosphate solution added, the solubility in solutions with little saline is estimated as greater than or equal to about 1 mg/ml.) The concentration of antigen in the solutions containing sodium chloride was much lower than could be accounted for by dilution of the stock solution with saline. In addition, the HPSEC analysis of solutions containing saline indicated that aggregated material was present in large proportions in those solutions containing salt (Table II).

TABLE 16-II

| HPSEC RESULTS FOR HEPATITIS A IN SALINE SOLUTIONS (DAY 1) | | |
|---|---|---|
| Sodium Chloride Concentration | Antigen (as monomer) | Aggregate:Monomer (AREA RATIO) |
| 0 (stock solution) | 106 mcg/ml | 0 |
| 0.15 N | 64 | 0.05 |
| 0.30 | 11 | 0.52 |
| 0.50 | 6.6 | 0.52 |

This indicated that the Hepatitis A was aggregating and then precipitating from solution even at concentrations below 50 mcg/ml when in saline. Figure 27 also shows the approximate composition of the product eluted from the ion-exchange column (represented as the point IEP). It is significantly above the levels of antigen that can be maintained in solution for even one day, and so without a significant alteration in the purification process it is necessary to dilute the product immediately after it elutes from the ion-exchange column. In order to minimize the dilution, and minimize the volume that will then be loaded onto the preparative size exclusion column the dilution should be done with 6 mM sodium phosphate to maintain nominal pH control while reducing the ionic strength and antigen concentration. A one to one dilution was estimated to decrease the antigen concentration to a point such that it was below the curve in Figure 27 represented by IEP∗ at about 25 mcg/ml and 150 mM sodium chloride in phosphate. Based on this data a dilution was implemented into the purification process.

Figure 28 shows the concentrations measured in the solutions described above at day 1 and day 14. It's apparent that the concentrations of antigen did drop over time between day 1 and day 14, and that one to one dilution does not dilute the antigen below the solubility curve, but only stabilizes the virus solution and serves to slow the onset of aggregation and precipitation. It is possible that further optimization of dilution will result in further enhancement of product yield.

Finally, as a check that the stock solution did contain Hepatitis A and not some other material that would co-elute on HPSEC the stock solution was subjected to SDS-PAGE and silver-stained. When compared with the SEC product the same protein bands were apparent.

EXAMPLE 17

HIGH PERFORMANCE SIZE EXCLUSION CHROMATOGRAPHY FOR MONITORING HEPATITIS A PURIFICATION SAMPLES

Introduction

A method has been developed to characterize and monitor process performance during purification of Hepatitis A. Analytical High Performance Size Exclusion Chromatography (HPSEC) with UV detection at 214 nm and 260 nm provides analysis of the relative amounts of the Hepatitis A virus and the key impurities (aggregate, nucleic acid-Hepatitis A adduct, and 660 kDa impurity) in process samples. In addition, HPSEC is used to quantitate the concentration of Hepatitis A in samples from the last 3 purification steps (extraction, ion-exchange and preparative SEC). This assay is used to monitor performance of the BENZONASE digestion and to provide a fingerprint of the Hep A impurity profile at each processing step for post run reference.

Hepatitis A process samples from 10 manufacturing lots have been assayed by HPSEC to provide process performance data for all stages of purification. The data from these analyses along with additional stability and spiking studies provides both a validation of the HPSEC assay and a clear indication of the Hepatitis A process consistency.

This Example summarizes both the validation data for the HPSEC assay, and the results of the analysis of 10 manufacturing lots. The report is organized into the following sections:

SECTION I        Instrumentation and Chromatographic Conditions A description of the HPSEC column and HPLC instrumentation and operating conditions.

SECTION II       Assay and Calibration Procedures A description of the preparation of calibration standards and assay procedures.

SECTION III      Linearity, Accuracy, and Reproducibility Statistical evaluation.

SECTION IV       Analysis of Results
A set of chromatograms from one lot is shown, along with a description of the type of data generated for each process sample and recommended alert limit.

SECTION V        Sample Storage Conditions
Storage studies were done for each type of process sample to determine stability at 4-6°C.

SECTION VI       Comparison of EIA and HPSEC Results

SECTION VII      Process Performance Consistency

APPENDIX A       Results of the HPSEC analysis of process samples from 10 lots.

SECTION I Instrumentation And Chromatographic Conditions

Materials And Reagents

CM 80 (MMD) phosphate buffered saline containing 6mM sodium phosphate 120 mM sodium chloride, pH 7.2

Instrumentation

(Equivalent substitution may be made except with the column)

| | |
|---|---|
| HPLC System: | RAININ Rabbit Pump equipped with 10 mL salt washing head. |
| Autosampler: | RAININ AI-1 equipped with recirculating chiller to maintain sample rack at 2-6 C |
| Detector: | RAININ UV-M two channel UV detector |
| Data acquisition system: | Dynamax HPLC Method Manager, Version 1.1 running on an Apple Macintosh SE |
| Column: | TosoHaas TSK PW4000x1, 7.8 x 300 mm L HPLC microvials - glass or polypropylene |

Chromatographic Conditions

| | |
|---|---|
| Column: | TosoHaas TSK PW4000x1, 7.8 x 300 mm L |
| Mobile Phase: | CM 80 (6mM sodium phosphate 120 mM sodium chloride, pH 7.2) |
| Flow rate: | 0.32 mL/min |
| Detection: | UV 214 nm and 260 nm @ 1V detector output |

| Injection volume: | 50 uL |
|---|---|
| Runtime between injections: | 55 minutes |

The TSK Pw4000x1 Column

The column used is a TSK PW4000x1, containing a methacrylate based Sum support. A calibration curve prepared from polyethylene oxide (PEO) standards is shown below. The dashed lines show the retention times of the 3 solutes of interest; the Hepatitis A aggregate, Hepatitis A, and the 660 kDa contaminant. Also shown by dashed lines are the retention times of 2 low molecular weight hydrophobic compounds, vitamin B12 and acetone. Both compounds lie to the right of the calibration curve; the retention of these compounds indicates the hydrophobic nature of this polymeric support. Retained peaks are observed in several of the process samples (See Figure 29). They generally elute near the salt peak and are presumed to be low molecular weight compounds.

SECTION II Assay And Calibration Procedures

Preparation Of Calibration Standards

A six level calibration curve is used for quantitation of Hepatitis A. Calibration standards are prepared by dilution of a purified Hepatitis A product. The SEC Product with a concentration of 12 ug/mL has been used as a standard. Six calibration standards covering the concentration range of 0.75 to 12 ug/mL are prepared by dilution of the SEC Product with CM80 as shown in the table below.

| Calibration Standard | Hep A Conc. (ug/mL) | uL Hep A | uL CM80 |
|---|---|---|---|
| 1 | 12 | 250uL 9854 SECProd | 0 |
| 2 | 9 | 180uL 9854 SECProd | 60 |
| 3 | 6 | 250uL 9854 SECProd | 250 |
| 4 | 3 | 250uL Cal Stan 3 | 250 |
| 5 | 1.5 | 250uL Cal Stan 4 | 250 |
| 6 | 0.75 | 250uL Cal Stan 5 | 250 |

The standards are prepared by addition of Hepatitis A solution and CM80 directly into the HPLC microvials. A calibration curve for Hepatitis A is prepared by plotting the log of the Hepatitis A peak area against the log of the concentration. A typical calibration curve is shown in Figure 30.

The log-log transformation of the calibration curve data is performed to reduce the dependence of the calibration curve upon the high concentration calibration standard. For the chromatography of biological macromolecules, the variance of the data tends to increase as the concentration increases. For HPLC calibration curves, the slope of the regression line is therefore primarily determined by the highest concentration standard, which is also the most variable.

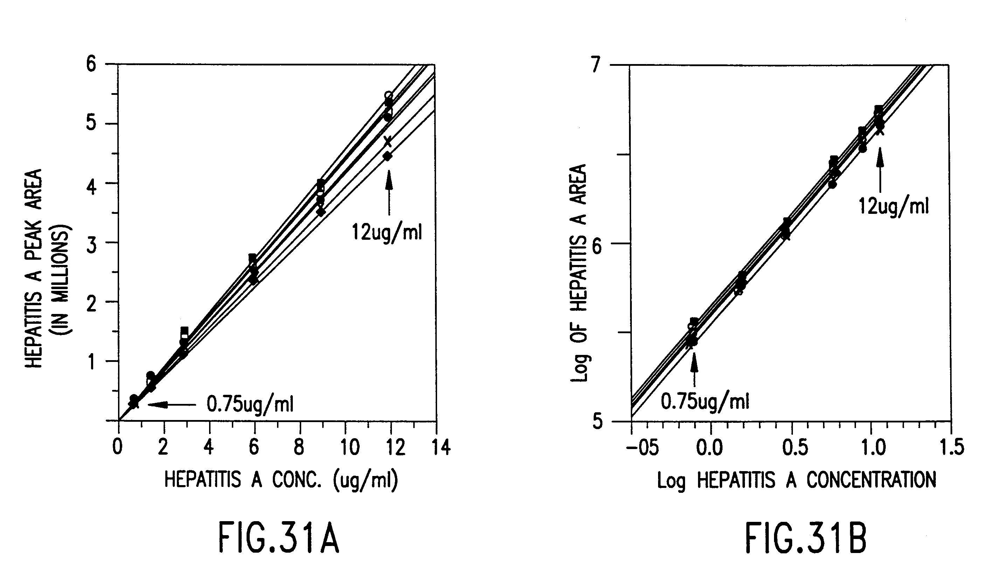

Graphs of 13 Hepatitis A calibration curves (prepared as described above over a 2 month time period) are shown in figure 31. On the left is the linear graph of the 13 curves, on the right the log-log transformation of the same 13 curves. The linear graph exhibits a pronounced "flaring" of the curves from a common origin, clearly demonstrating the increasing variance of the Hepatitis A peak area as concentration increases. The result is inter-curve variation in the slope values.

A log-log transformation of these calibration curves makes the variance (RSD) constant across the entire concentration range. The result is reduced inter-curve slope variation, as shown in figure 31B.

Sample Preparation Procedures

A. Hepatitis A Process Samples:

Process samples from the following steps are assayed:
1. Filtered Lysate

2. Nuclease Treated Filtered Lysate

3. PEG Precipitate Resuspended

4. Chloroform Extracted Aqueous Phase

5. Anion Exchange Product

6. Size Exclusion Product

The Filtered Lysate, Nuclease Treated Filtered Lysate, and the Size Exclusion Product samples should not be diluted prior to injection. The PEG Precipitate Resuspended sample should be centrifuged (10,000 x g for 5 min) to remove remaining particulates prior to injection. The PEG Precipitate Resuspended sample is diluted 1:4 (v/v) with CM80 prior to injection. The Chloroform Extracted Aqueous Phase and the Anion Exchange Product are assayed undiluted and assayed diluted 1:1 (v/v) with CM80 prior to injection to bring the concentration to within the range of the calibration curve. For all samples, the assay is performed in triplicate and results of the three assays are averaged. Samples are stored in the autosampler at 2-6 C while awaiting injection.

Triton is weakly retained on the TSK PW4000 x1 column, and additional eluate is required to remove it. Therefore samples containing Triton (Filtered Lysate, and Nuclease Treated Filtered Lysate) should be followed by injection of a CM80 blank to insure removal prior to injection of additional samples. Samples may be run in reverse order beginning with the SEC product to avoid late eluting interference peaks.

Assay Procedure

1. Inject 50 ul of each of the six calibration standards. The retention time of Hepatitis A should be 19.0 +/- 1 minute.

2. Prepare samples as described above. Inject 50 uL. Each sample is assayed in triplicate.

SECTION III Linearity, Accuracy And Reproducibility

Thirteen calibration curves covering the range of 0.75 to 12.0 ug/ml were prepared on 13 different days during a 5 week time period. The regression equation obtained for each curve was used to calculate the concentration of each of the calibration standards comprising that curve. The results calculated from the 13 curves compared to the nominal concentration for these 6 calibration standards are shown below in Table 1.

TABLE 17-1

| Comparison Of Measured To Nominal Concentrations Of Pure Hepatitis A. Calculations Based On Regression Line. | | | | | | |
|---|---|---|---|---|---|---|
| NOMINAL CONCENTRATIONS OF HEPATITIS A CALIBRATION STANDARDS | | | | | | |
| Calibration Curve (Date: mm/dd) | 12ug/ml | 9ug/ml | 6ug/ml | 3ug/ml | 1.5ug/ml | 0.75ug/ml |
| Curve 1 (10/6) | 11.91 | 8.93 | 6.03 | 3.03 | 1.54 | 0.73 |
| Curve 2 (10/7) | 11.88 | 8.88 | 6.00 | 3.04 | 1.52 | 0.74 |
| Curve 3 (10/8) | 12.26 | 8.81 | 5.95 | 3.00 | 1.49 | 0.76 |
| Curve 4 (10/10) | 11.97 | 9.04 | 6.00 | 3.04 | 1.45 | 0.76 |
| Curve 5 (10/13) | 11.89 | 8.97 | 6.06 | 3.02 | 1.52 | 0.74 |

| | | | | | | |
|---|---|---|---|---|---|---|
| Curve 6 (10/14) | 11.75 | 9.08 | 6.07 | 3.06 | 1.47 | 0.75 |
| Curve 7 (10/20) | 11.94 | 8.98 | 5.96 | 2.98 | 1.60 | 0.72 |
| Curve 8 (10/21) | 11.99 | 8.95 | 5.99 | 3.01 | 1.53 | 0.74 |
| Curve 9 (10/22) | 11.98 | 8.94 | 5.90 | 2.97 | 1.45 | 0.70 |
| Curve 10 (10/26) | 12.08 | - | 6.00 | 2.98 | 1.48 | 0.76 |
| Curve 11 (10/28) | 12.01 | 9.05 | 6.00 | 2.95 | 1.51 | 0.75 |
| Curve 12 (11/18) | 11.48 | 9.23 | 6.14 | 3.00 | 1.52 | 0.73 |
| Curve 13 (12/3) | 11.88 | 8.90 | 6.10 | 3.04 | 1.50 | 0.74 |
| AVERAGE | 11.92 | 8.98 | 6.02 | 3.01 | 1.51 | 0.74 |
| Stan.Deviation | 0.17 | 0.10 | 0.06 | 0.03 | 0.04 | 0.02 |
| RSD | 0.01 | 0.01 | 0.01 | 0.01 | 0.03 | 0.03 |

Between day reproducibility is very good for all six calibration levels, with RSD values of less than 3% over the entire concentration range. Accuracy is also very good; for the 12, 9, 6, 3, 1.5ug/ml and 0.75 ug/ml standards all the measured concentrations were within 5% of the nominal concentration.

Spiking Study

To further assess the specificity and accuracy of the HPSEC assay, a spiking experiment was performed. The Anion Exchange Product and Chloroform Extracted Aqueous Phase samples were spiked with an equal volume of SEC Product from the same lot. The results are shown in Table 2 below. All samples were assayed in triplicate.

TABLE 17-2

| Recovery Of Hepatitis A Spiked Into Process Samples. | | | |
|---|---|---|---|
| SAMPLE | Measured Concentration (ug/ml) | Theorical Concentration (ug/ml) | % Spike Recovered |
| Chloroform Extracted. Aq. | 7.27 | | |
| Anion Exchange Prod. | 8.87 | | |
| SEC Product | 5.40 | | |
| SPIKED SAMPLES | | | |
| Chloro.Ex.Aq. 1:1 w/SEC | 6.40 | 6.33 | 101 |
| AnionEx.Prod. 1:1 w/SEC | 7.18 | 7.14 | 101 |

For both samples, complete spike recovery was found, indicating specificity and accuracy for HPSEC concentration determinations.

SECTION IV Analysis Of Results

Summary

As a series, the HPSEC chromatograms provide a picture of the Hepatitis A purification process. Comparison of the Filtered Lysate to the Nuclease Treated Lysate provides a confirmation of BENZONASE activity. For these samples, chromatograms at both 214 nm and 260 nm are obtained and integrated.

Comparison of the last 4 process samples, PEG Pellet Resuspended, Chloroform Extracted Aqueous Phase, the Anion Exchange Product and the SEC Product shows clearance of the 2 main process contaminants; aggregated material and the 660 kDa protein contaminant. Calculation of the Hepatitis A area percent for these samples provides assurance that each process step is functioning as designed to remove these contaminants. Finally, the HPSEC assay is used to calculate the concentration of Hepatitis A in the final 3 process steps.

Samples of Filtered Lysate, Nuclease Treated Lysate, PEG Pellet Resuspended, Chloroform Extracted Aqueous Phase, Anion Exchange Product and SEC Product from 10 Hepatitis A lots manufactured in MMD have been assayed by HPSEC. The chromatographic profile for each process sample has been very reproducible across these 10 lots.

In this section, a chromatogram for each of the 6 process samples is shown, along with a description of the information obtained from each sample, and typical results of a triplicate assay.

Filtered Lysate and Nuclease Treated Lysate

Chromatograms for Filtered Lysate and Nuclease Treated Lysate samples from each lot were obtained at wavelengths of 214 nm and 260 nm. For each of these samples, the chromatographic profile obtained at 214 nm is completely different than the profile obtained at 260 nm. Comparative analysis of these chromatograms reflects the purpose of the HPSEC assay of these samples: to find a quantifiable difference between the Filtered Lysate and Nuclease Treated Lysate that will indicate the completeness of the BENZONASE digestion. Therefore in this section the profiles of the Filtered Lysate and Nuclease Treated Lysate are compared; first at 214 nm, then the same comparison at 260 nm. The characteristic data obtained from each sample is presented, as is a recommendation for quantifying the completeness of BENZONASE activity.

Filtered Lysate and Nuclease Treated Lysate at 214 nm

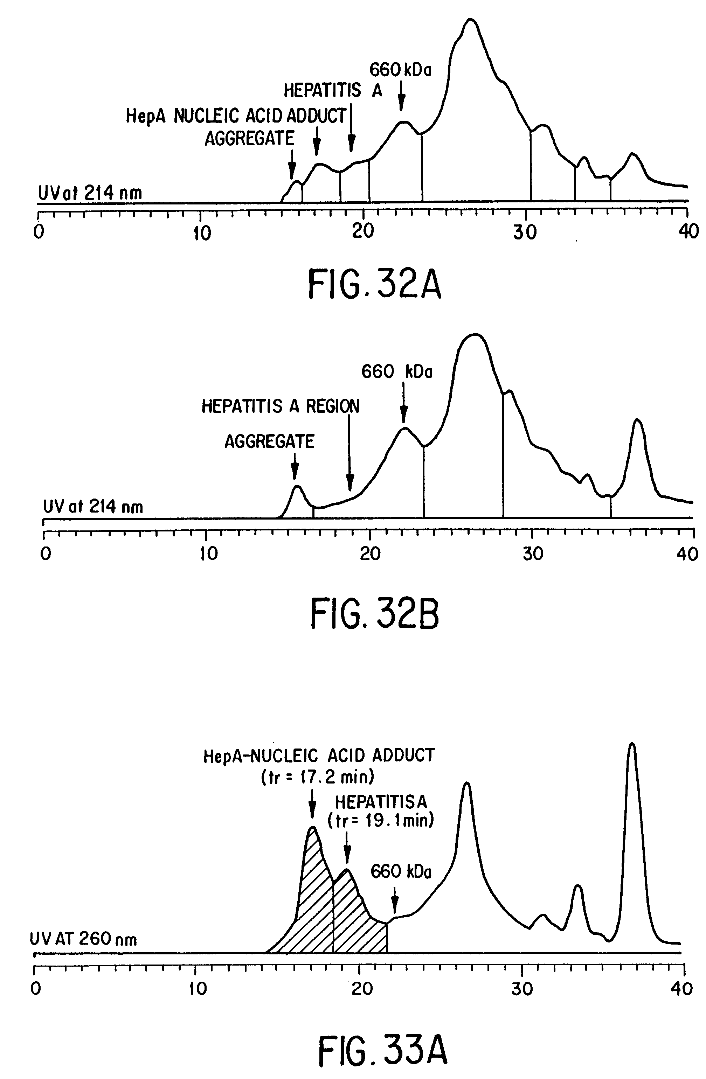

The chromatograms of the Filtered Lysate and Nuclease Treated Lysate at 214 nm are shown in Figure 32. At 214 nm, both samples show a series of partially resolved peaks. Because of the low resolution obtained for these samples, integration of individual peaks in the Hepatitis A region is impossible; the only information obtainable from these samples at 214 nm is the total UV area for all peaks. The results of the triplicate analysis of the samples are also shown in Figure 32. This total $UV_{214\ nm}$ area does not include the retained peak at 36 minutes. The total $UV_{214\ nm}$ area of the Nuclease Treated Lysate sample is 15% lower than the Filtered Lysate sample as a result of BENZONASE digestion; and a visual comparison of the 2 chromatograms (Figure 32) does show a loss of absorbance in the Hepatitis A region. However, a definitive measurement of this digestion is impossible due to the low resolution of the peaks involved. The total UV214 nm area has been obtained for 9 lots, these values are given in Appendix A. These total $UV_{214\ nm}$ areas do not provide any direct or unique process performance information, their use has been solely in monitoring sample stability, therefore calculating total $UV_{214\ nm}$ area is not necessary for routine reporting.

| (A) FILTERED LYSATE | | |
| --- | --- | --- |
| Filtered Lysate | | Total UV Area 214 nm (uV) |
| 1 | | 27,248,238 |
| 2 | | 26,404,744 |
| 3 | | 26,782,947 |
| | Average | 26,811,976 |
| | StanDev | 344,966 |

| (B) NUCLEASE TREATED LYSATE | | |
| --- | --- | --- |
| Nuclease Treated Lysate | | Total UV Area 214 nm (uV) |
| 1 | | 22,728,263 |
| 2 | | 22,841,564 |
| 3 | | 22,784,419 |
| | Average | 22,784,749 |
| | StanDev | 46,256 |

Filtered Lysate And Nuclease Treated Lysate At 260 nm

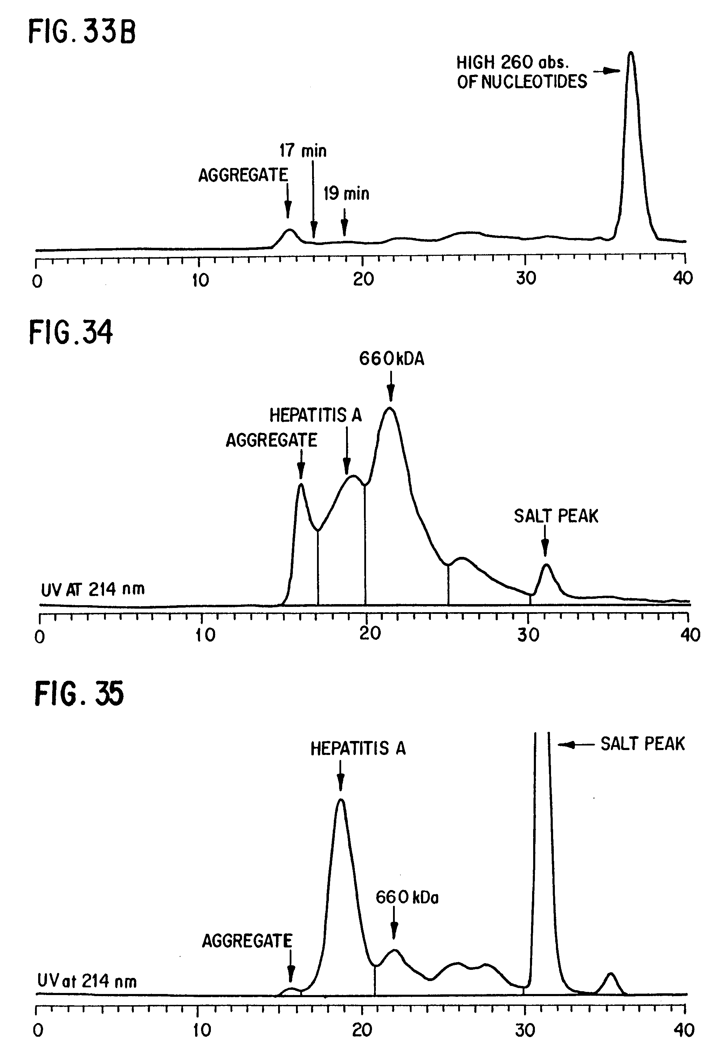

The chromatograms of the Filtered Lysate and Nuclease Treated Lysate at 260 nm are shown in Figure 33. At 260 nm the Filtered Lysate shows a more distinct profile, with 5 to 6 resolved peaks. The first two peaks, with retention time of 17.2 and 19.1 minutes, comprise the Hepatitis A-nucleic acid adduct and Hepatitis A. Since both peaks have some degree of nucleic acids associated with them, they dominate the 260 nm HPSEC profile.

Comparison of the Filtered Lysate sample to the Nuclease Treated Lysate at 260 nm (Figures 33A and 33B) provides a clear and unambiguous confirmation of the BENZONASE activity. The prominent peaks apparent in the Filtered Lysate sample have been completely removed by the BENZONASE digestion, Figure 33B shows no discernible peaks at 17 to 19 minutes.

Confirmation of BENZONASE Digestion

Analysis of the Filtered Lysate and Nuclease Treated Lysate samples from 9 lots are shown below in Table 17-3. The $UV_{260\ nm}$ area for 17 and 19 minute peaks for these samples shows that BENZONASE addition results in the complete removal of these peaks for all lots tested. Therefore for this sample, the recommended criteria to confirm BENZONASE activity would be reduction of the 17+19 minute peak $UV_{260\,nm}$ in Nuclease Treated Lysate to less than 10% of that in the Filtered Lysate.

TABLE 17-3

| Reduction of $UV_{260\,nm}$ area by BENZONASE for 9 lots. | | | |
|---|---|---|---|
| Lot # | FILTERED LYSATE (A) AREA17+19min Peaks (260 nm) | NUCLEASE TREATED LYSATE (B) AREA17+19min Peaks (260 nm) | RESIDUAL A260 (B/A x 100) |
| 984 | 1,240,987 | 0 | 0 |
| 985 | 947,988 | 0 | 0 |
| 1047 | 1,085,719 | 0 | 0 |
| 102 | 708,663 | 0 | 0 |
| 101 | 831,179 | 0 | 0 |
| 108 | 1,060,441 | 0 | 0 |
| 100 | 895,005 | 0 | 0 |
| 139 | 1,377,974 | 0 | 0 |
| 236 | 1,281,608 | 0 | 0 |
| Average= | 1,018,495 | 0 | 0 |
| StanDev= | 205,034 | | |

| FIGURE 33.(A) FILTERED LYSATE | | | |
|---|---|---|---|
| Filtered Lysate | Area of 17+19 min Peaks(UV) | | Total UV Area(UV) 260nm |
| 1 | | 1,192,597 | 3,193,055 |
| 2 | | 1,259,216 | 3,034,891 |
| 3 | | 1,271,147 | 3,094,820 |
| | Average | 1,240,987 | Average | 3,107,589 |
| | StanDev | 34,562 | StanDev | 65,198 |

## FIGURE 33 (B)   NUCLEASE TREATED LYSATE

| Nuclease Treated Lysate | Area of 17+19 min Peaks(UV) |
|---|---|
| 1 | 0 |
| 2 | 0 |
| 3 | 0 |

Average and StanDev = 0

PEG Pellet Resuspended Sample

Shown below in Figure 34 is the chromatogram of the PEG Pellet Resuspended sample at 214 nm. The characteristic profile of this sample consists of several partially resolved peaks; aggregated material, the Hepatitis A peak, the 660 kDa contaminant, other low molecular weight contaminants, and the salt peak.

The Hepatitis A area percent is calculated for this sample, (Hep A Peak Area/Total Peak Area x 100), the total area does not include the salt peak. Results for the triplicate analysis of the PEG Pellet Resuspended sample are shown below.

| PEG Pellet Res. | Hep A Area Percent | |
|---|---|---|
| 1 | | 25.9 |
| 2 | | 27.0 |
| 3 | | 27.4 |
| | Average | 26.8 |
| | StanDev | 0.7 |

The typical profile for this sample has the 660 kDa contaminant as the major component, with the amount of aggregate being the most variable of the components. The amount of aggregate in the typical lot is lower than that seen. Results of the HPSEC analysis of the PEG Pellet Resuspended samples from 10 lots are shown in Table A2 of Appendix A. The Hepatitis A area percent values range from 18.5% to 32.0%; all were successfully purified by the following process steps.

Therefore stringent alert limit are not necessary for this sample; certainly area percent values of greater than 18% are acceptable, however the effect of having lower area percent values will not be known until they are encountered in the Hepatitis A process.

Chloroform Extracted Aqueous Phase Sample

The chromatogram for the Chloroform Extracted Aqueous Phase sample from is shown below in Figure 35. The Hepatitis A peak is the major peak in this sample (excluding the salt peak) and it is well resolved from the 660 kDa contaminant and small amounts of aggregate.

For this sample, the Hepatitis A concentration is determined, and the Hepatitis A area percent is calculated. The area percent calculation does not include the salt peak. Results of the triplicate analysis of the Chloroform Extracted Aqueous Phase sample are shown below.

| Chloro.Extracted Aq. | Hepatitis A Conc ug/ml) | | Hepatitis A Area Percent | |
|---|---|---|---|---|
| 1 | | 28.64 | | 61.0 |
| 2 | | 29.76 | | 62.8 |
| 3 | | 30.65 | | 61.2 |
| | Average | 29.68 | Average | 61.7 |
| | StanDev | 0.82 | StanDev | 0.8 |

Results of the HPSEC analysis of the Chloroform Extracted Aqueous Phase samples from 10 lots are shown in Table A2 of Appendix A. The Hepatitis A area percent values for full lots ranged from 60.0 to 72.8%; the two half-lots were significantly higher, with area percent values of 79.4 and 85.2% respectively. The Hepatitis A concentrations were more variable, ranging from 14.4 to 29.7 ug/ml. The variability of the Hepatitis A concentrations may be related to the variation in upstream sample volumes through the consistency lots.

Comparison of the HPSEC profile of the PEG Pellet (Figure 34) to the Chloroform Extracted Aqueous Phase (Figure 35) provides a clear indication of the importance of this extraction step to the Hepatitis A process; this step selectively removes large amounts of the 660 kDa contaminant without significant yield loss of Hepatitis A. Although the 660 kDa contaminant can be removed in the two subsequent chromatography steps, in both cases if the 660 kDa is the major component of the feed solutions peak shaving strategies are required that will lower Hepatitis A yields. In addition, extensive optimization experiments have indicated that the extraction efficiency is quite sensitive to changes in mixing times (see Example 15). The Hepatitis A area percent value for the Chloroform Extracted Aqueous Phase provides quantitative in-process verification of successful extraction and impurity removal.

Therefore, an alert limit of Hepatitis A area percent values of >60% is recommended for the Chloroform Extracted Aqueous Phase sample. Area percent values below 60% should be viewed as potentially indicative of processing problems, (such as with the process mixing apparatus), particularly if this occurs regularly.

Anion Exchange Product

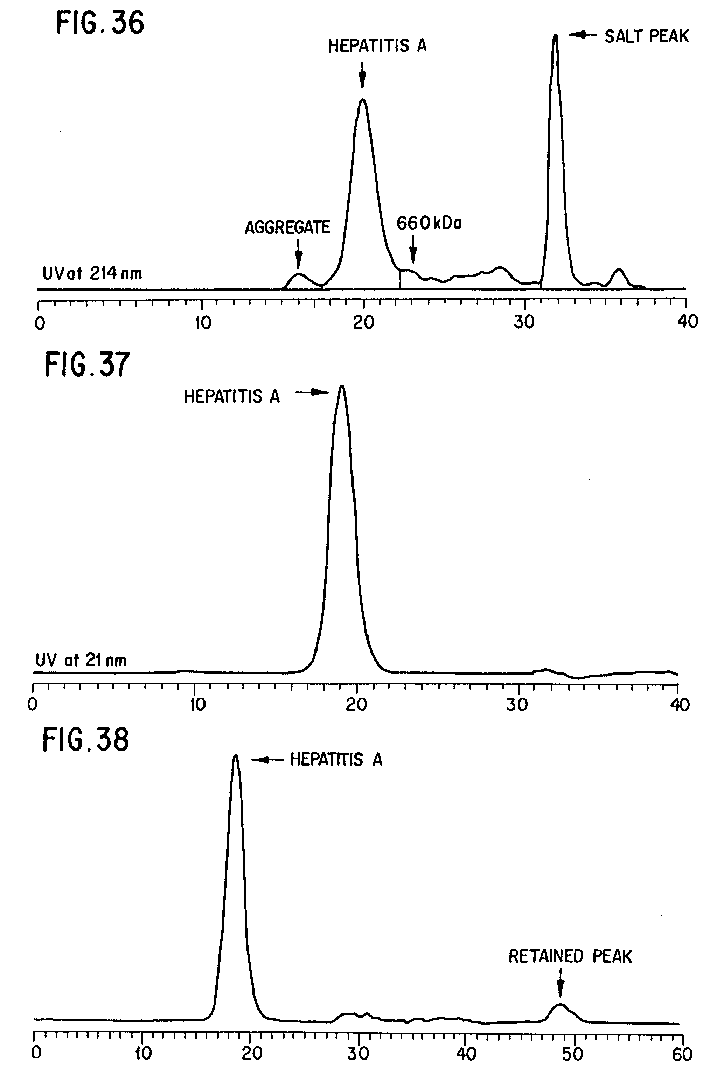

Shown in Figure 36 is the Anion Exchange Product sample. The Hepatitis A peak is the major component of this sample, with small amounts of aggregate, 660 kDa contaminant, and low molecular weight contaminants also present.

For this sample, the Hepatitis A concentration is determined, as well as the Hepatitis A area percent, excluding the salt peak and the retained peak. Results of the triplicate analysis of the Anion Exchange Product sample from Rx2009854 is shown below.

| An.Ex.Prod | Hepatitis A Conc. (ug/ml) | | Hepatitis A Area Percent | |
|---|---|---|---|---|
| 1 | | 31.98 | | 82.8 |
| 2 | | 32.63 | | 81.4 |
| 3 | | 32.37 | | 81.6 |
| | Average | 32.32 | Average | 81.9 |
| | StanDev | 0.27 | StanDev | 0.6 |

Results of the HPSEC analysis of the Anion Exchange Product sample from 10 lots is shown in Table A2 of Appendix A. The area percent values obtained range from 78.6 to 87.4% for full lots, with half-lot values greater than 90%. The increased area percent values found for the half-lots is not surprising considering the increased purity of the feed for these lots.

Concentration values ranged considerably, (over 2 fold) for these lots; this variation, like that observed for the extraction step, is probably due to variation in sampling for these lots.

In the Hepatitis A process, the anion exchange chromatography functions as a polishing step; it removes some of the 660 kDa contaminant and other low MW compounds, but the purity of the Anion Exchange Product is dependent on the purity of the feed for this step, it cannot remove large amounts of the 660 kDa without yield loss due to peak shaving. Therefore, this step cannot compensate for a failure in upstream processing. Typically, the Hepatitis A area percent increases by 10-20% across the anion exchange chromatography step, the higher the feed purity the higher the resulting product purity. For this sample, recommended alert limit would be area percent values of >75% and an increase in area percent of > 10% across this step. When both criteria are met, the process and anion exchange step are both functioning as developed. Failure to achieve an area percent value of >75% by itself is possible for the anion exchange step if the upstream steps fail, failure to achieve an increase in area percent of >12% would likely indicate a process problem very specific to the anion exchange step, such as a peak collection malfunction or column channeling.

Size Exclusion Chromatography (SEC) Product

The SEC Product profile shows highly purified Hepatitis A, as shown in Figure 37 for the SEC Product.

For this sample, the concentration of Hepatitis A is determined. Results of the duplicate analysis of the SEC Product are shown below.

| Sample Assay | | Hepatitis A Conc. (ug/ml) |
| --- | --- | --- |
| 1 | | 17.34 |
| 2 | | 16.34 |
| | Average | 16.84 |
| | StanDev | 0.50 |

Results of the HPSEC analysis of the SEC Products from 10 lots is shown in Table A2 of Appendix A. For this sample, area percent values have not been calculated. The profile shown in Figure 37 clearly shows a high purity product, free of detectable amounts of aggregate and 660 kDa contaminant. However, assay of the SEC Products over a 60 minute period has shown the presence of a small retained peak at about 49 minutes as shown in Figure 38. The size of this peak varies from lot to lot, with the lot shown (Figure 38) having the highest amount observed to date. Identification of this retained peak should be made before calculation of area percent values for this sample is indicative of its purity. Until this identification is made, area percent values for this sample will only include peaks between the excluded and included volumes (retention times of 16-32 minutes). With these restrictions, area percent values for all lots tested to date are 100%.

## SECTION V Sample Stability

In process samples have proven to be stable up to several months as measured by HPSEC and EIA. Small losses may be observed at the ion exchange step, generally less than 10% over a period of two weeks. However, following this initial decline, even these samples remain stable. In addition, the final SEC product is stable for periods up to at least one year.

## SECTION VI Comparison of EIA and HPSEC Results

EIA and HPSEC results obtained for 3 samples (the Chloroform Extracted Aqueous Phase, Anion Exchange Product and SEC Product) from 11 lots (Lots 2 through 12) were compared.

A graph showing the log of the HPSEC concentration plotted against the log of the EIA concentration for these 33 samples is shown below in Figure 45. A regression line fit to this data gives a slope of 0.88 and an coefficient of correlation (r) of 0.94. The slope value approaching 1 and the r value of 0.94 indicates a very good correlation exists between the two assays, especially considering the inherent variability of each measurement.

## SECTION VII Process Performance Consistency

HPSEC analysis of lots 2 through 14 reveals a very stable and reproducible process. Based on the extensive HPSEC analysis of in-process samples taken from batches produced in manufacturing, the purification process provides excellent reproducibility for clearance of major contaminants. Figure 46 provides one measure of this, where the area % monomeric virus has been plotted for batches. This measure of stream composition for the last four steps of the purification shows great consistency across these batches. Lots 10 and 11, which show higher purity at the extraction and anion exchange steps, are both half-size lots.

EXAMPLE 17: APPENDIX A

## TABLE 17-A1

Data summary for Lysate and Nuclease Treated Lysate Samples. All samples were assayed in triplicate, and results of the three assays averaged.

| FILTERED LYSATE SAMPLES | | | |
|---|---|---|---|
| LOT # | TOTAL AREA (214nm) | TOTAL AREA (260nm) | AREA17+19min (260nm) |
| 1 | 26,811,976 | 3,107,589 | 1,240,987 |
| 2 | 22,032,023 | 2,722,501 | 947,988 |
| 3 | 24,322,218 | 2,696,138 | 1,085,719 |
| 4 | 19,934,567 | 1,588,166 | 708,663 |
| 5 | 23,633,378 | 1,657,484 | 831,179 |
| 6 | 26,311,501 | 2,590,678 | 1,060,441 |
| 7 | 20,336,561 | 2,225,138 | 895,005 |
| 8 | 27,019,891 | 3,195,019 | 1,377,974 |
| 9 | 25,671,116 | 2,804,591 | 1,281,608 |
| Average= | 23,800,264 | 2,472,839 | 1,018,495 |
| StdDev= | 2,648,361 | 565,612 | 205,034 |

| NUCLEASE TREATED LYSATE SAMPLES | | |
|---|---|---|
| LOT # | TOTAL AREA (214nm) | AREA17+19m in (260nm) |
| | | 0 |
| 1 | 22,784,749 | 0 |
| 2 | 21,417,391 | 0 |
| 3 | 22,658,768 | 0 |
| 4 | 17,766,458 | 0 |
| 5 | 17,984,377 | 0 |
| 6 | 23,280,554 | 0 |
| 7 | 17,231,064 | 0 |
| 8 | 23,218,865 | 0 |
| 9 | 21,797,999 | 0 |
| Average= | 20,904,469 | |
| StdDev= | 2,491,146 | |

## APPENDIX A (CONTINUED)

## TABLE 17-A2

Data summary for PEG Pellet Resuspended, Chloroform Extracted Aqueous Phase, Anion Exchange Product, and SEC Product samples. All samples were assayed in triplicate, and results of the three assays averaged.

| LOT # | PEG RES Area Percent | CHLOROFORM EX. AQ. Area Percent | Conc. (ug/ml) | DIL. ANION EX.PROD Area Percent | Conc. (ug/ml) | SEC PRODUCT Area Percent | Conc. (ug/ml) |
|---|---|---|---|---|---|---|---|
| 2 | 29.6 ± 0.6 | 64.7 ± 1.7 | 22.25 ± 0.35 | 80.6 ± 0.1 | 28.46 ± 0.21 | 100 | 10.01 ± 0.09 |
| 3 | 26.8 ± 0.7 | 61.7 ± 0.8 | 29.68 ± 0.82 | 81.9 ± 0.6 | 32.32 ± 0.27 | 100 | 16.84 ± 0.50 |
| 4 | 32.0 ± 2.3 | 64.7 ± 0.4 | 23.72 ± 0.01 | 83.7 ± 0.9 | 29.12 ± 0.48 | 100 | 14.57 ± 0.18 |
| 5 | 31.2 ± 2.6 | 60.0 ± 0.2 | 25.89 ± 0.26 | 78.6 ± 0.3 | 35.74 ± 0.89 | 100 | 18.22 ± 0.75 |
| 6 | 25.9 ± 0.3 | 67.1 ± 1.3 | 15.90 ± 0.26 | 82.9 ± 0.3 | 16.39 ± 0.09 | 100 | 7.96 ± 0.18 |
| 7 | 29.4 ± 0.9 | 66.7 ± 1.6 | 19.10 ± 0.30 | 84.2 ± 0.3 | 15.73± 0.26 | 100 | 7.80 ± 0.20 |
| 8 | 29.2 ± 0.7 | 70.8 ± 1.1 | 16.40 ± 0.10 | 87.4 ± 0.5 | 20.01 ± 0.07 | 100 | 9.68 ± 0.12 |
| 9 | 20.7 ± 1.4 | 72.8 ± 3.4 | 14.32 ± 0.28 | 85.7 ± 0.5 | 14.61 ± 0.05 | 100 | 8.05 ± 0.01 |
| 10 | 23.7 ± 0.1 | 85.2 ± 1.3 | 10.02 ± 0.06 | 97.0 ± 0.3 | 10.24 ± 0.04 | 100 | 6.24 ± 0.06 |
| 11 | 18.5 ± 0.7 | 79.4 ± 0.5 | 7.32 ±0.42 | 90.5 ± 1.0 | 8.62 ± 0.10 | 100 | 5.24± 0.00 |
| *Half-Size Lots | | | | | | | |

EXAMPLE 18

Clinical Study Results

The purified vaccine of this invention has been administered to healthy humans. No serious vaccine related adverse effects have been identified.

The efficacy of a single 25-unit dose of the vaccine was demonstrated in children in the Monroe study, the results of which were published in the August 13, 1992 edition of the New England Journal of Medicine, [Werzberger et al., NEJM 327 (no.7);453-457, (August 13, 1992)]. The differences between the Monroe study material and the production material, both of which form part of this invention, are highlighted in figure I, and both of which are contrasted with the Phase I/II study material produced in roller bottles and harvested by mechanical means, with the attendant limitations on scale of production implied in that process. A 25 unit dose (25 ng HAV protein) of the purified preparation of hepatitis A virus of this invention was sufficient to achieve over 95% seroconversion of children and adolescents 2-16 years of age within 4 weeks of immunixation. This same dose has been shown to provide 100% protection in children and adolescents beginning as early as 21 days after receipt of a single dose of vaccine.

Weight and age have now been identified as response variables in adults. In one month after a single 25 unit dose, 83% of adults ($\geqq$ 18 years) and 97% of children and adolescents (2 to 17 years) seroconvert. Two doses of 25 units given two weeks apart increases the seroconversion rate in adults to 93%. Seroconversion seven months after a second or third 25 unit dose results in greater than 99% seroconversion in both age groups. Persistence in seropositivity is about 100% up to 12 months and 36 months in both age groups. Thus, it is clear that a single 25 unit dose is sufficient to achieve seroconversion in at least 80% of any recipient population within one month of immunization. This data is summarized in table 18-1 (regimen indicates 25 unit dose given at 0 and 24 weeks or 0, 2, and 24 weeks; GMT = geometric mean anti-HAV antibody titer). Immunization with a larger dose induces seroconversion in a higher percentage of recipients.

Table 18-1:

| Regimen | Age | Seroconversion at Indicated Week after Initial Immunization | | |
|---|---|---|---|---|
| | | 4 | 24 | 28 |
| 0 & 24 | 18-29 | 83% (100/120) | 87% (33/38) | 100% (24/24) |
| GMT-responders | | 21 | 41 | 2132 |
| 0, 2, 24 | 18-29 | 98% (115/118) | 100% (33/33) | 100% (24/24) |
| GMT-responders | | 33 | 90 | 4379 |
| 0 & 24 | 30-89 | 72% (265/370) | 80% (111/138) | 100% (71/71) |
| GMT-responders | | 22 | 34 | 1332 |
| 0, 2, 24 | 30-89 | 85% (321/380) | 89% (118/132) | 100% (75/75) |
| GMT-responders | | 28 | 59 | 1796 |

In addition to the above data, a cohort of patients found to be seronegative at 4 weeks (23 cases total) were retested at 24 weeks and elicitation of an anamnestic response (>10 fold increase in antibody titer) at 28 weeks. Two individuals showed an approximate six fold increase in titer post six month dose. The remaining 21 individuals, 91%, showed seroconversion and an anamnestic response. Thus, these individuals are occult seroconverters and are likely to be protected by immunization with the vaccine of this invention, even though apparently seronegative for an extended period post-immunization.

**Claims**

1. A commercial scale process for the preparation of hepatitis A virus of greater than 95% purity with respect to protein by SDS polyacrylamide gel electrophoresis and silver stain analysis, which comprises the steps of:

a) culturing hepatitis A virus in a large quantity in a cell sheet in a large surface area static surface reactor (SSR);

b) removing the hepatitis A virus from the cell sheet culture by detergent permeation of the infected cell sheet such that the hepatitis A virus is liberated from the cell culture;

c) optionally removing non-hepatitis A virus specific nucleic acids at this stage from the harvested hepatitis A virus, by nuclease treatment;

d) concentrating the hepatitis A virus removed from the cell culture, by membranes and diafiltration, or capture by ion exchange;

e) removing non-hepatitis A virus specific proteins from the concentrated hepatitis A virus of step (d), by a combination of organic extraction, PEG precipitation, ion exchange, including removal of contaminating free nucleic acid if not previously completed in step c), and gel permeation chromatography steps; and

f) recovering the purified hepatitis A virus from step (e).

2. A purified hepatitis A virus obtained by the process of Claim 1.

3. The process of Claim 1 wherein:
step a) comprises inoculation with hepatitis A virus derived from the supernatant of an HAV infected SSR into a static surface reactor in which MRC-5 cells are cultured in cell sheets, and culture medium is constantly circulated through the static surface reactor and a loop in which reactor control, aeration and replenishment of culture components is accomplished.

4. The process of Claim 1 wherein:
step b) comprises harvest of hepatitis A virus by treatment of a hepatitis A virus infected cell sheets with between about a 0.05% to 1% solution of Triton-X 100 to liberate the hepatitis A virus.

5. The process of Claim 1 wherein:
step c) comprises digestion with BENZONASE.

6. A process for commercial scale production of an inactivated hepatitis A virus vaccine, which is greater than 95% pure HAV on the basis of protein, which comprises:

(a) Culturing a large quantity of hepatitis A virus (HAV) in NUNC CELL FACTORIES, COSTAR CUBES, or STATIC SURFACE REACTORS (SSRs), including an SSR comprising regular mesh elements upon which cells are able to grow at high density per unit volume, in which a cell sheet of MRC-5 cells has been established;

(b) Harvesting the cultured HAV by removal of the culture medium and addition of a harvest solution containing 0.1% Triton X-100;

(c) Treating the harvested HAV with BENZONASE to digest contaminating free nucleic acids;

(d) Concentrating the BENZONASE treated HAV by capture on an ion exchange column, and eluting the captured HAV with about 0.35 M NaCl, followed by precipitation with polyethylene glycol;

(e) Resuspending the PEG precipitated HAV and extracting the concentrated HAV with chloroform/isoamyl alcohol (24:1,v/v) with vigorous agitation, and separating and retaining the aqueous phase;

(f) Chromatographing the aqueous phase from the organic extraction on DEAE TOYOPEARL 650M anion exchange matrix at about 90 to 150 mM NaCl, and eluting the HAV with a gradient up to about 1 M NaCl, and diluting the pooled peak HAV fractions to a salt concentration below about 150 mM and an HAV concentration below about 20 μg/ml such that HAV precipitation is minimized or eliminated;

(g) Collecting the peak HAV fractions from step f) and chromatographing the HAV on tandem size exclusion columns of TOYOPEARL HWSSS AND HW65S;

(h) Inactivating the gel filtered HAV by treatment with formalin at a concentration of 1/1000 for five days, followed by sterile filtration, adsorption to or coprecipitation with aluminum hydroxide, and dispensation into unit doses equivalent to 25 -100 ng per dose of inactivated purified HAV.

7. The use of the product of the process of Claim 6 for the preparation of a medicament for preventing HAV infection.

8. The HAV vaccine prepared according to the method of Claim 6.

9. A process for culturing HAV which comprises growing the HAV in a COSTAR CUBE or STATIC SURFACE REACTOR, optionally including regular mesh elements of titanium or stainless steel gauze.

**10.** The process of Claim 9 which comprises producing between about 5,000 and 50,000 doses of a 25 ng per dose vaccine of inactivated HAV of greater than 95% pure HAV from a single COSTAR CUBE culture vessel.

**11.** A purified preparation of hepatitis A virus wherein greater than 95% of the protein is hepatitis A virus specific, and less than 7% of the protein mass of the preparation is non-HAV nucleic acid or lipid and between about 0-180% of the preparation is carbohydrate composed of glucose.

**12.** The preparation of Claim 11 wherein the hepatitis A virus is formalin inactivated.

**13.** The preparation of Claim 12 formulated as a 25-100 ng dose of HAV on the basis of protein with about 100-1000 µg aluminum hydroxide.

**14.** A purified preparation of hepatitis A virus having the following characteristics, wherein each characteristic is reported on a per µg of protein basis, and the method in parenthesis was used for determination of the characteristic:
a) lipid content (gas chromatography): < 0.1µg;
b) carbohydrate detected as glucose 0.1 - 2.5µg;
non-glucose carbohydrate < 0.1µg;
(TFA hydrolysis, Dionex)
c) hepatitis A virus specific protein > O.95µg;
(amino acid analysis and western blot),
d) hepatitis A virus specific RNA 0.1-0.2µg
e) contaminating DNA < 0.001µg.

**15.** The preparation of Claim 14 wherein the hepatitis A virus is formalin inactivated.

**16.** The preparation of Claim 15 adsorbed onto aluminum hydroxide.

**17.** A purified preparation of inactivated hepatitis A virus such that a single 25 unit dose, equivalent to about 25 ng of hepatitis A virus protein based on amino acid analysis, is sufficient to achieve 95% or greater seroconversion of children and adolescents 2-16 years of age within 4 weeks of immunization.

**18.** A purified preparation of inactivated hepatitis A virus such that between one and three 25 to 100 unit doses, equivalent to about 25 ng to 100 ng of hepatitis A virus based on amino acid analysis, is sufficient to achieve 80-95% seroconversion of a recipient population within 4-28 weeks of immunization.

**19.** The process of Claim 1 which comprises culture of hepatitis A virus in a static surface reactor, and a suitable culture medium is replenished and removed at a rate between about 0.010 and 0.3 mL/cm²/day.

**20.** The process of Claim 1 which comprises culture of hepatitis A virus in a static surface reactor, and the culture medium is replenished and removed at a constant rate.

**21.** A hepatitis A virus vaccine having the following nominal composition per 25 -100 unit/0.1-1 mL dose:

EP 0 583 142 A2

| COMPONENT | AMOUNT |
|---|---|
| Sodium Chloride | 900-9000 μg |
| Aluminum | 50-500 μg |
| sodium tetraborate | 3.8-38 μg |
| Formaldehyde | < 0.4 μg |
| Glucose (polymer) | 0-1000 ng |
| Protein | 25-100 ng |
| Chloroform | <44 ng |
| HAV RNA | 2-20 ng |
| Non-glucose carbo. | 0-20 ng |
| Fatty acid | <20 ng |
| Neomycin | <4 ng |
| MRC-5 DNA | <0.02 ng |
| Bovine serum albumin | <0.1 ng |
| BENZONASE | <0.0001 ng |

22. A hepatitis A virus vaccine having the following nominal composition per 25 unit/0.5 mL dose:

| COMPONENT | AMOUNT |
|---|---|
| Sodium Chloride | 4,500 μg |
| Aluminum | 250 μg |
| sodium tetraborate | 19 μg |
| Formaldehyde | < 200 ng |
| Glucose (polymer) | <20 ng |
| Protein | 25 ng |
| Chloroform | <22 ng |
| HAV RNA | 5 ng |
| Non-glucose carbo. | <4 ng |
| Fatty acid | <1 ng |
| Neomycin | <1 ng |
| MRC-5 DNA | <0.005 ng |
| Bovine serum albumin | <0.02 ng |
| BENZONASE | <0.00002 ng |

23. A purified preparation of inactivated hepatitis A virus such that a single 25 unit dose, equivalent to about 25 ng of hepatitis A virus protein based on amino acid analysis, is sufficient to achieve 95% or greater protection of children and adolescents 2-16 years of age as early as 21 days after immunization.

47

| PHASE I/II | MANUFACTURING | MONROE |
|:---:|:---:|:---:|
| RB | SSR | NCF |
| ↓ | ↓ | ↓ |
| MECHANICAL | TRITON | TRITON |
| ↓ | ↓ | ↓ |
| METHYLENE CHLORIDE | NUCLEASE | Conc/Diaf XAD |
| | ↓ | |
| | CAPTURE | |

PEG PRECIPITATION

↓

CHCl₃ EXTRACTION

↓

ION EXCHANGE

↓

GEL FILTRATION

FIG.1

FIG.2A

FIG.2B

FIG.2C

FIG.2D

FIG.3

FIG.4

FIG.11

50

FIG. 5

FIG. 6A

FIG. 6B

FIG. 7

FIG.8

FIG.10

FIG.12

FIG.9B

GUR (ug/REACTOR-d) x $10^4$

GAUZE

SOLID

TIME (d)

FIG.9A

GUR (ug/cm$^2$-d)

GAUZE

SOLID

TIME (d)

FIG.13B

GLUCOSE (mg/dl)

MEDIUM REFEED

MOI=1

MOI=10

MOI=0

TIME POST-INFECTION (d)

FIG.13A

CELL DENSITY ($10^4$/cm$^2$)

MOI=0

MOI=10

MEDIUM REFEED

MOI=1

TIME POST-INFECTION (d)

56

FIG.14

FIG. 15

FIG.16

EP 0 583 142 A2

FIG.17A

FIG.17B

FIG. 18A

FIG. 18B

FIG.19

FIG.20

FIG.22

FIG.21

FIG.23

FIG.24

EP 0 583 142 A2

FIG.25

FIG.26

64

FIG.27

FIG.28

FIG.29

FIG.30

FIG.31B

FIG.31A

FIG. 32A

FIG. 32B

FIG. 33A

## FIG. 33B

## FIG. 34

## FIG. 35

FIG. 36

HEPATITIS A

SALT PEAK

AGGREGATE

660 kDa

UV at 214 nm

0          10          20          30          40

FIG. 37

HEPATITIS A

UV at 21 nm

0          10          20          30          40

FIG. 38

HEPATITIS A

RETAINED PEAK

0      10      20      30      40      50      60

70

logHPSEC conc.=0.8832∗log EIA conc.+0.1347

FIG.39

FIG. 40